# FASCICULE DE BREVET EUROPEEN

(11) **EP 4 262 738 B1**
(45) Date de publication et mention de la délivrance du brevet: **19.02.2025**
(21) Numéro de dépôt: 21814793.2
(22) Date de dépôt: 23.11.2021
(51) Int. Cl.: A61K 8/9789, A61Q 19/08, A61Q 19/00, A61K 36/82, A61K 8/60, A61K 8/55, A61K 8/81

(54) **PROCEDE D'OBTENTION D'EXTRAITS AQUEUX DE FEUILLES DE THES, COMPOSITIONS COMPRENANT DE TELS EXTRAITS ET LEURS UTILISATIONS COSMETIQUES**
VERFAHREN ZUR GEWINNUNG VON WÄSSRIGEN EXTRAKTEN AUS TEEBLÄTTERN, ZUSAMMENSETZUNGEN MIT SOLCHEN EXTRAKTEN UND KOSMETISCHE VERWENDUNGEN DAVON
METHOD FOR OBTAINING AQUEOUS EXTRACTS OF TEA LEAVES, COMPOSITIONS COMPRISING SUCH EXTRACTS AND COSMETIC USES THEREOF

(30) Priorité: 23.11.2020 FR 2012019
(43) Date de publication de la demande: 25.10.2023
(73) Titulaire: ISP Investments LLC, Wilmington, Delaware 19805 (US)
(72) Inventeur: MOURET, Laura, 06410 BIOT (FR); OGER, Elodie, 06600 ANTIBES (FR)
(74) Mandataire: Miquel, Corinne
(86) Numéro de dépôt international: PCT/EP2021/082631
(87) Numéro de publication internationale: WO 2022/106718

(56) Documents cités:
- EP-A2- 0 995 427
- WO-A1-2009/063068
- WO-A1-2020/089831
- WO-A1-2021/156104
- WO-A2-00/45769
- DE-A1- 19 820 629
- DE-A1- 19 824 727
- US-A1- 2010 119 463
- ABRUTYN E: "Chelating Agents", COSMETICS & TOILETRIES MAGAZINE, ALLURED PUBLISHING CORP, US, vol. 126, no. 8, 1 August 2011 (2011-08-01), pages 542, 544 - 546, XP007919732, ISSN: 0361-4387
- DATABASE WPI Week 201683, 1 August 2016 Derwent World Patents Index; AN 2016-48336W, XP002804038, FU Q; SHENG J; SHI W; XU H: "Pu'er tea gel used for preventing and treating atopic dermatitis, is prepared using pu'er tea extract, matrix, humectant, co-solvent, preservative, and distilled water"
- PINGJING ZHANG ET AL: "Use of small RNA as antiaging cosmeceuticals", JOURNAL OF COSMETIC SCIENCE, 1 November 2013 (2013-11-01), United States, pages 455 - 468, XP055282728, Retrieved from the Internet <URL:http://journal.scconline.org//pdf/cc2013/cc064n06/p00455-p00468.pdf> [retrieved on 20190726]

## Description

### Domaine technique

L'invention concerne le domaine de la cosmétique et plus particulièrement des ingrédients actifs d'origine naturelle entrant dans la préparation de formulation cosmétique pour améliorer l'aspect de la peau ou la protéger.

L'invention concerne un procédé d'obtention d'extraits aqueux de différents types de thés et en particulier de thé frais, de thé pu'er, de thé matcha ou de thé vert, ainsi que des extraits de thé enrichis en petits ARN, en sucres, en composés phénoliques, en acides organiques, en théine et en théanine, les compositions cosmétiques comprenant de tels extraits et leurs utilisations cosmétiques pour le soin de la peau, du cuir chevelu et des phanères et plus particulièrement pour protéger la peau des agressions externes et de l'oxydation, lutter contre les signes du vieillissement cutané, augmenter la photoprotection, éclaircir la peau et améliorer l'hydratation de la peau.

### Arrière-plan technique de l'invention

Les plantes du genre *Camellia* comportent un grand nombre d'espèces différentes appartenant à la famille des Théacées. Les botanistes estiment qu'il existe entre 100 et 250 espèces de plantes appartenant à ce genre. Le genre *Camellia* est originaire d'Asie Orientale et méridionale de l'est depuis la chaîne himalayenne, au Japon et en Indonésie.

Parmi les espèces les plus connues et utilisées se trouve l'espèce *Camellia sinensis,* communément appelée plante à thés qui comprend elle-même trois variétés botaniques : *Camellia sinensis var. assamica, Camellia sinensis var. sinensis* (Yunnan) et *Camellia sinensis var. Cambodiensis.* Une autre espèce *Camellia assamica* est connue en tant que plante à thé mais sa culture plus délicate limite son utilisation.

L'espèce *Camellia sinensis* est utilisée en industrie agro-alimentaire pour la production de différents thés en utilisant les feuilles. Le traitement des feuilles de *Camellia sinensis* combiné aux conditions de culture donneront au thé sa particularité. Les thés se différencient notamment par leur couleur et leurs caractéristiques organoleptiques.

Une fois récoltées, les feuilles subissent un traitement spécifique du type de thé et qui détermine le degré d'oxydation final des feuilles. Les différentes étapes clefs sont le flétrissage (assouplissement des feuilles de thé dans le but de les faire sécher), le roulage (pour en extraire les huiles essentielles), la fixation (par chauffage afin de stopper l'oxydation des feuilles), le séchage (courant d'air chaud afin d'abaisser le taux d'humidité) et la fermentation (les feuilles sont placées dans un environnement pauvre en oxygène afin d'accélérer la prolifération des micro-organismes).

On définit le thé vert dans la présente demande comme un thé *(Camellia sinensis)* dont l'oxydation naturelle est stoppée rapidement après la cueillette des feuilles. Afin d'obtenir du thé vert, les feuilles de théier sont flétries, roulées pour en extraire les sucs puis chauffées afin de bloquer le processus d'oxydation. Il existe deux méthodes de chauffage possibles : la méthode chinoise qui utilise des bassines de cuivre placées sur un feu ou la méthode japonaise passant les feuilles à travers des jets de vapeur (permettant ainsi de préserver leurs propriétés). Le blocage de l'oxydation permet de maximiser la teneur en molécules intéressantes comme la catéchine et la théanine.

On définit le thé matcha dans la présente demande, comme un thé vert du Japon qui a été obtenu à partir de feuilles de thé (*Camellia sinensis*) réduites en poudre (matcha signifie « poudre de thé »). Pour obtenir du thé matcha les producteurs pratiquent la culture en zone ombragée afin de freiner la photosynthèse et de ralentir ainsi la transformation de la théanine en tanins. Cette technique donne au thé matcha une saveur unique végétale et fruitée. Les feuilles sont chauffées à la vapeur pour stopper l'oxydation et obtenir un thé vert. Une fois séchées, les feuilles sont broyées avec une meule en pierre pour obtenir une poudre très fine vert vif. Le thé matcha est très riche en polyphénols, une classe d'antioxydants aux bienfaits reconnus pour la santé.

On définit dans la présente demande comme thé pu'er, un thé vert post-fermenté appelé aussi pu'er sheng en Asie ou pu'er brut ou encore raw pu'er en occident. Le thé pu'er est originaire de la région du Yunnan en Chine. Il est fabriqué à partir de feuilles de l'espèce *Camellia sinensis var. assamica.* Les feuilles de théier subissent une première oxydation sur le lieu de production avant d'être pressées sous forme de galettes ou de briques. Les galettes subissent alors une fermentation modérée par l'intermédiaire de micro-organismes, fermentation qui peut être accélérée en milieu humide. Par la suite les galettes de thé sont partiellement oxydées pendant le processus de vieillissement naturel.

De nombreux extraits de feuilles de thé sont décrits dans la littérature en partie pour leur richesse en composés phénoliques souvent les polyphénols de type catéchine. En revanche, il existe peu d'extraits sur le marché ou décrits dans l'art antérieur qui revendiquent une composition riche en différents types de phytomolécules autres que les polyphénols. En effet, la plupart des méthodes d'extraction de feuilles de thé de l'art antérieur décrivent des procédés utilisant des solvants organiques pour leurs capacités à extraire les polyphénols et principalement les polyphénols de type catéchine (WO2006/111666, KR2016021734A, KR2018125828A). Les extraits décrits sont riches en polyphénols et flavonoïdes, mais ne contiennent pas de molécules phénoliques de type acide phénolique car ces derniers ne sont pas extraits par ce type de technique. En effet les acides phénoliques sont principalement extraits dans un solvant polaire et idéalement avec de l'eau. Aussi les autres phytocomposés, qui sont reconnus pour leurs effets bénéfique tels que les acides aminés, les acides organiques, les protéines, des sucres et des oligo-ribonucléotides tels que les petits ARN ne sont pas optimalement extraits par ces méthodes.

Les utilisateurs de produits cosmétiques souhaitent maintenant utiliser des formules aussi naturelles que possibles et qui présentent une efficacité aussi importante voire meilleure que les produits synthétiques. Dans la présente invention les extraits réalisés sont 100 % naturels afin de répondre aux exigences des consommateurs.

De plus, malgré les nombreux produits cosmétiques anti-âge déjà sur le marché, le besoin de nouveaux ingrédients cosmétiques efficaces d'origine naturelle est de plus en plus fort.

Un problème que se propose de résoudre l'invention est de fournir de nouveaux extraits aqueux de thé répondant à l'exigence du marché cosmétique actuel concernant les critères de naturalité et présentant néanmoins une efficacité biologique remarquable, ainsi qu'une absence de toxicité.

En effet, une trop forte concentration en composés phénoliques tels que la catéchine peut provoquer des phénomènes de phototoxicité et limiter la stabilité de l'extrait dans le temps. Les extraits décrits dans la présente invention ont une concentration en polyphénols et en autres phytomolécules d'intérêt compatible avec une bonne efficacité. Ils présentent également une stabilité dans le temps importante et une absence de toxicité.

Un autre problème que se propose encore de résoudre l'invention est de fournir de nouveaux extraits aqueux de thé enrichis en composés connus pour leur efficacité sur la peau comme les sucres, les composés phénoliques, les acides organiques, la théine et la théanine ainsi que des petits ARN obtenus par le procédé.

Les inventeurs ont développé des nouveaux extraits de thés spécifiquement enrichi en petits ARN, sucres, en composés phénoliques et organiques, en acides organiques, en théine, en théanine, par un procédé ne présentant pas les inconvénients des procédés de l'art antérieur, comme par exemple l'utilisation de détergents et de solvants potentiellement toxiques en cosmétique.

Les extraits ainsi obtenus peuvent être utilisés en cosmétique pour le soin de la peau, du cuir chevelu et des phanères, pour obtenir de multiples bénéfices et plus particulièrement pour protéger la peau des agressions externes et de l'oxydation, lutter contre les signes du vieillissement cutané, augmenter la photoprotection, éclaircir la peau, améliorer l'hydratation de la peau, renforcer la fonction barrière ou encore apaiser la peau.

### Résumé de l'invention

L'invention a pour premier objet un procédé d'obtention d'un extrait aqueux de feuilles de thé, de l'espèce *Camellia sinensis,* choisies parmi le thé frais, le thé pu'er, le thé matcha ou le thé vert, comprenant les étapes suivantes :
a) on met en présence les feuilles de thé ou la poudre de thé avec de l'eau, puis éventuellement on les broie ;
b) on ajoute de l'acide phytique ;
c) on ajuste le pH à une valeur comprise entre 10 et 11 ;
d) on maintien le mélange sous agitation pendant au moins une heure à une température comprise entre 40 et 80°C ;
e) on purifie le mélange obtenu en c) pour éliminer la matière végétale solide résiduelle et récolter le filtrat ;
f) on ajuste le pH à une valeur comprise entre 6 et 8 ;
g) on traite par du charbon actif en poudre ;
h) on filtre pour éliminer le charbon actif et on récolte le filtrat ;
i) on traite le filtrat à l'aide de polyvinylpolypyrrolidones (PVPP) ;
j) on filtre pour éliminer les polyvinylpolypyrrolidones et récolter le filtrat ;
k) on contrôle et réajuste le pH du filtrat, si nécessaire, à une valeur comprise entre 6 et 6,5.

L'invention a pour deuxième objet un extrait brut aqueux de feuilles de thé frais, de thé pu'er, de thé matcha ou de thé vert, enrichi en petits ARN d'une longueur d'au maximum 150 nucléotides, en sucres, en composés phénoliques et en acides organiques, dépourvu d'ADN, susceptible d'être obtenu par le procédé de l'invention, ayant un poids sec de 5 à 30 g/kg, et comprenant de 0,5 à 10 g/kg de sucres, de 0,050 à 2 g/kg d'acides organiques, de 0,050 à 5 g/kg de composés phénoliques dont 0 à 200 mg/kg de catéchine, 0,020 à 2 g/kg de théine et 0,020 à 2 g/kg de théanine et de 50 à 350 mg/kg d'ARN de petit poids moléculaire d'une longueur d'au maximum 150 nucléotides.

L'invention a encore pour objet un extrait aqueux dilué de feuilles de thé frais, de thé pu'er, de thé matcha ou de thé vert, enrichi en petits ARN d'une longueur d'au maximum 150 nucléotides, en sucres, en composés phénoliques et en acides organiques, dépourvu d'ADN, susceptible d'être obtenu par le procédé de l'invention, ayant un poids sec compris entre 4 à 20 g/kg de poids sec, une concentration de 0,2 à 5 g/kg de sucres, 0,030 à 1 g/kg d'acides organiques, 0,030 à 3 g/kg de composés phénoliques dont 0 à 120 mg/kg de catéchine, 0,010 à 1 g/kg de théine et 0,010 à 1 g/kg de théanine et 10 à 250 mg/kg d'ARN de petit poids moléculaire d'une longueur d'au maximum 150 nucléotides, en poids du poids total de l'extrait.

L'invention a pour troisième objet une composition comprenant, en tant qu'ingrédient actif, une quantité efficace d'au moins un extrait aqueux dilué de feuilles de thé choisi parmi un extrait de thé frais, de thé pu'er, de thé matcha ou de thé vert selon l'invention, ou tout mélange de ceux-ci, et un milieu physiologiquement acceptable.

L'invention a pour quatrième objet l'utilisation cosmétique de la composition selon l'invention pour le soin de la peau, du cuir chevelu et des phanères et plus particulièrement pour protéger la peau des agressions externes et de l'oxydation, lutter contre les signes du vieillissement cutané, augmenter la photoprotection, éclaircir la peau, ou encore améliorer l'hydratation de la peau.

### Brève description des dessins

L'invention et les avantages qui en découlent seront mieux compris à la lecture de la description et des modes de réalisation non limitatifs qui suivent, illustrés au regard des figures annexées dans lesquelles :
[Fig. 1] Evaluation de la teneur en sucres totaux par méthode colorimétrique dans les différents extraits de thé obtenus selon les exemples 1 et 2.
[Fig. 2] Evaluation de la teneur en polyphénols par méthode colorimétrique dans les différents extraits de thé obtenus selon les exemples 1 et 2.
[Fig. 3] Evaluation de la teneur en acides organiques par HPLC-MS dans les différents extraits de thé obtenus selon les exemples 1 et 2.
[Fig. 4] Evaluation de la teneur en théanine, théine et catéchine par HPLC-UV dans les différents extraits de thé obtenus selon les exemples 1 et 2.
[Fig. 5] Evaluation de l'inhibition de la hyaluronidase des différents extraits de thé obtenus selon les exemples 1 et 2 par mesure de la turbidité.
[Fig. 6] Evaluation des niveaux d'espèces oxygénées réactives dans des biopsies de peau stressées par du glutamate monosodique et traitées avec les différents extraits de thé pu'er obtenus selon les exemples 1 et 2.

### Description détaillée de l'invention

### Définitions

Tous les termes utilisés dans la présente description ont le sens le plus largement connus sauf mention contraire. Pour les besoins de l'invention les termes suivants sont définis comme suit :
On entend par « extrait de thé » tous les types de traitement de feuilles de l'espèce *Camellia sinensis.*

On entend par « thé » les feuilles de l'espèce *Camellia sinensis* sous toutes leurs formes ; entières ou en poudre, sèches ou fraîches.

Par « petits ARN » ou « ARN de petits poids moléculaires », ou « petits ARN d'une longueur d'au maximum 150 nucléotides » on entend des ARN (acides ribonucléiques) non codants, de petit poids moléculaires, d'une longueur d'au maximum 150 nucléotides, tels que tous types de petits ARN non messagers, simples et/ou doubles brins, par exemple les micro-ARN, les ARN interférents, les introns, les petits ARN nucléaires ou encore tout fragment d'ARN provenant de la plante et extraits par le procédé décrit dans l'invention. Dans les extraits selon l'invention les petits ARN sont sous la forme d'un mélange complexe de nombreux ARN ayant une taille majoritairement comprise entre 25 et 150 nucléotides. Ainsi, les ARN issus de synthèses et donc non naturels, ne rentrent pas dans cette définition.

Par « acides organiques » on entend les acides α-hydroxylés (ou AHA), c'est-à-dire des acides carboxyliques dérivés de sucres contenus dans les feuilles de thé, tels que les acides lactique, malique, citrique, tartrique, succinique et uronique.

Par « composés phénoliques », on entend les molécules d'origine végétale qui possèdent au moins un cycle aromatique de type phénolique portant un ou plusieurs groupements hydroxyles, tels que les acides phénoliques, les flavonoïdes telles que la catéchine ou ses dérivés, les tanins ou tout autre polyphénol. Les composés phénoliques sont reconnus pour être de puissantes molécules antioxydantes, aussi bien dans la plante que pour une utilisation cosmétique. Ces métabolites secondaires de la plante sont également produits dans les mécanismes de défense de la plante par des stress biotique ou abiotique.

Par « sucres » on entend tous les types de sucres présents dans les plantes, tels que les monosaccharides comme le glucose, le fructose ainsi que les oligo et polysaccharides. En règle générale, les polysaccharides contiennent plus de dix unités monosaccharides, tandis que les oligosaccharides contiennent de trois à dix unités monosaccharide.

Par « phytomolécules d'intérêt », on entend l'ensemble des molécules présentes dans les extraits de thé de l'invention et notamment, les petits ARN d'une longueur d'au maximum 150 nucléotides, les sucres, les composés phénoliques, les acides organiques, la théine et la théanine.

Quand une gamme de valeur est décrite, les bornes de cette gamme doivent être comprises comme incluant explicitement toutes les valeurs intermédiaires de la gamme. Par exemple, une gamme de valeur comprise entre 1 % et 10 % doit être comprise comme incluant 1 %, 2 %, 3 %, 4 %, 5 %, 6 %, 7 %, 8 %, 9 %, et 10 %, ainsi que toutes les valeurs décimales entre 1 % et 10 %.

Les valeurs numériques en pourcentage sont des pourcentages en poids, c'est-à-dire le poids d'un composé par rapport au poids total du mélange envisagé, sauf spécifié autrement.

Les compositions décrites dans la présente demande peuvent « comprendre », « consister en » ou « consister essentiellement en » les composés essentiels ou les ingrédients optionnels.

« Consister essentiellement en » signifie que la composition ou le composant peut inclure des ingrédients additionnels, mais seulement si les ingrédients additionnels ne modifient pas les caractéristiques de base ou les nouvelles caractéristiques de la composition ou de l'utilisation décrite dans la présente demande.

Par « application topique » on désigne le fait d'appliquer ou d'étaler l'extrait selon l'invention, ou une composition le contenant, à la surface de la peau ou d'une muqueuse.

« Peau » désigne la peau du visage, notamment le contour des yeux et la bouche, le nez, le front, le cou, les mains, mais aussi la peau de l'ensemble du corps.

« Cuir chevelu » désigne la peau recouvrant le crâne, incluant les follicules pileux et les espaces cutanés inter-folliculaires.

« Phanères » désigne chez les cheveux et les poils (produits par les follicules pileux, ainsi que les ongles, riches en kératine.

« Eclaircir la peau » signifie diminuer l'intensité de la couleur de la peau liée au contenu en mélanine de l'épiderme, soit de manière homogène, soit de manière localisée en agissant sur des désordres pigmentaires, tels que les tâches de senescence ou lentigos séniles.

Par « quantité efficace » on désigne la quantité minimum d'extrait selon l'invention qui est nécessaire pour obtenir au moins une des activités biologiques recherchées ou pour protéger la peau des agressions externes et de l'oxydation, lutter contre les signes du vieillissement cutané, augmenter la photoprotection, éclaircir la peau et améliorer l'hydratation de la peau, sans que cette quantité soit toxique.

Par « hydratation de la peau », on entend le contenu et la répartition en eau des couches supérieures de l'épiderme.

**On entend** par « amélioration de l'hydratation cutanée », toutes améliorations des modifications de l'aspect extérieur de la peau dues à la déshydratation comme, par exemple, la sécheresse, les tiraillements et l'inconfort, que cet état soit lié à des facteurs internes ou externes, tels que des conditions environnementales défavorables.

Par « signes du vieillissement cutané » on entend toutes modifications de l'aspect extérieur de la peau dues au vieillissement comme, par exemple, les rides et ridules, les crevasses, les poches sous les yeux, les cernes, le flétrissement, la perte d'élasticité, de fermeté et/ou de tonus de la peau, mais également toutes modifications internes de la peau qui ne se traduisent pas systématiquement par un aspect extérieur modifié comme, par exemple, l'amincissement de la peau, ou toutes dégradations internes de la peau consécutives à des stress environnementaux tels que la pollution et les rayonnements solaires incluant les UV.

Par « signes du vieillissement cutané » on entend également les désordres pigmentaires tels que le lentigo sénile ou lentigo solaire.

Par « agressions externes » on entend les rayonnements solaires, incluant la lumière visible, les rayonnements UV et infrarouges, la pollution, pouvant provenir de l'atmosphère ambiante à l'extérieur ou à l'intérieur des habitations et incluant des particules de différentes tailles (10 µm pour les PM10, 2,5 µm pour les PM 2,5, ou inférieur à 100 nm pour les particules ultrafines) ainsi que plusieurs éléments chimiques (composés organiques volatils, hydrocarbures aromatiques polycycliques, métaux lourds...).

On entend par « améliorer l'aspect de la peau » que le grain de la peau apparaît plus fin, la luminosité plus intense et le teint plus homogène.

Par « physiologiquement acceptable » on entend un milieu ou un solvant adapté pour une mise en contact avec les couches externes de la peau ou des muqueuses, sans toxicité, irritation, réponse allergique indue et similaire ou réaction d'intolérance, et proportionné à un rapport avantage/risque raisonnable.

Il est bien entendu que l'invention concerne les mammifères et plus particulièrement l'être humain.

### Procédé d'extraction

Les protocoles d'extraction des acides ribonucléiques (ARN) classiquement décrits utilisent des solvants inadaptés à un usage cosmétique (Zumbo, P. 2014 "Phenol-chloroform Extraction", 2014). Ces méthodes visent à obtenir des acides nucléiques (ARN ou ADN ou petits ARN) complètement purifiés, c'est-à-dire exempts de toute autre molécule d'intérêt tels que les métabolites secondaires, vitamines, sucres, peptides, etc. qui peuvent avoir des effets bénéfiques pour la peau et présentent donc un intérêt cosmétique.

On connait également le document FR2831168 qui décrit un procédé d'obtention d'un extrait de plante riche en acides nucléiques (ADN et ARN). Le procédé utilise des enzymes cellulolytiques.

On connait encore de l'art antérieur les documents de brevet EP1723958 et WO03101376 qui décrivent une composition pour application topique comprenant un oligonucléotide d'ARN double brins de synthèse d'une longueur de 12 à 40 nucléotides, de séquence connue ayant une fonction de siARN (« short interfering »).

On connait encore le document FR 1502361 (également publié sous le numéro WO2017084958) qui décrit un procédé d'obtention d'un extrait aqueux de plantes, enrichi en acides ribonucléiques (ARN) de petit poids moléculaire, pour préparer des compositions cosmétiques. Le procédé utilise de l'EDTA à une concentration comprise entre 2 et 15 mM.

L'utilisation d'acide phytique au lieu de l'EDTA présente les avantages suivants :
Contrairement à l'EDTA, l'acide phytique est une molécule naturelle présente dans l'enveloppe des graines comme les céréales et les légumineuses. De ce fait, l'utilisation d'acide phytique permet d'obtenir un extrait de thé d'origine 100 % naturelle tout en maintenant une bonne efficacité d'extraction des phytomolécules contenues dans la plante. L'efficacité d'extraction des petits ARN ainsi que celles des autres composés présents dans les feuilles de thé, tels que les sucres, les composés phénoliques, ou des acides organiques comme l'acide tartrique, malique ou citrique.

L'invention a ainsi pour premier objet un procédé d'extraction mis en œuvre pour l'obtention d'un extrait aqueux de feuilles provenant de tous types de thé de l'espèce *Camellia sinensis.*

Plus particulièrement l'invention concerne un procédé d'extraction mis en œuvre pour l'obtention d'un extrait aqueux de feuilles de thé frais, de thé pu'er, de thé vert ou encore de thé matcha.

Le procédé d'extraction de l'invention permet d'obtenir un extrait riche en phytomolécules d'intérêt cosmétique tels que les petits ARN d'une longueur d'au maximum 150 nucléotides, les sucres, les composés phénoliques, les acides organiques, la théine et la théanine en évitant l'utilisation de solvants qui ne sont pas considérés comme des solvants cosmétiques.

Le procédé de l'invention présente un impact réduit sur l'environnement.

Le procédé de l'invention peut s'appliquer à des feuilles de thé fraîches ou à des feuilles de thé ayant subi différentes préparations pour répondre à l'appellation de thé matcha, thé vert ou thé pu'er.

Dans une première étape a) du procédé, on mélange les feuilles de thé avec de l'eau. L'eau utilisée est une eau distillée, déminéralisée ou encore une eau riche en sels minéraux et/ou oligo-éléments. L'eau préférentiellement utilisée est de l'eau distillée.

Les feuilles de thé peuvent se présenter sous différentes formes : fraîches, sèches, entières ou réduites en poudre.

Préférentiellement, les feuilles de thé fraîches ou sèches sont broyées dans de l'eau dans l'étape a). Le broyage des feuilles de thé est une action mécanique qui permet une meilleure extraction. Le broyage est inutile pour les feuilles de thé déjà sous forme de poudre, comme le thé matcha, qui est directement mélangé à l'eau.

A l'étape a), le rapport matière végétale / eau est de 3 % à 20 % en poids/poids, plus préférentiellement de 3 à 10 %.

Dans une étape b) on ajoute l'acide phytique au mélange thé-eau de l'étape a).

Le procédé d'extraction continue avec une lyse alcaline en présence d'acide phytique favorisant la complète déstructuration des membranes cellulaires ainsi que de la membrane nucléaire.

L'acide phytique qui est un excellent agent chélatant, va entrainer la fragilisation et la destruction des membranes pecto-cellulosiques des cellules végétales en séquestrant par complexation les ions divalents tels que les ions calcium qui forment des ponts ioniques entre les molécules de pectines entourant les microfibrilles de cellulose. Ceci a pour conséquence de favoriser la libération du contenu cellulaire au cours de l'extraction. L'étape de traitement par l'acide phytique en milieu basique est essentielle pour enrichir l'extrait en ARN de petits poids moléculaires et également assurer un meilleur rendement d'extraction des autres phytomolécules d'intérêt ; à savoir les sucres, les composés phénoliques, les acides organiques ainsi que la théine et la théanine.

L'acide phytique est une molécule naturellement présente dans l'enveloppe des graines, comme les céréales et les légumineuses. L'acide phytique est présent sous forme de sels de calcium ou parfois de magnésium, et il joue un important rôle pour la plante, par exemple, c'est la source principale de phosphore.

De manière préférée, l'acide phytique utilisé est une poudre d'acide phytique sous forme de sel de sodium. Préférentiellement on l'utilise à une concentration comprise entre 1 et 10 mM, préférentiellement entre 1 et 5 mM et plus préférentiellement encore à une concentration de 3 mM.

La concentration de 3 mM est également optimale pour avoir un meilleur rendement des autres composés d'intérêt comme les sucres, les composés phénoliques, les acides organiques, la théine et la théanine.

A l'étape c) on ajuste le pH à une valeur basique comprise entre 10 et 11 par l'ajout de soude (NaOH). Lors de l'étape c) il est essentiel que le pH soit basique, compris entre 10 et 11. De préférence, le pH est ajusté à une valeur comprise entre 10,3 et 10,8. En effet, ce niveau de pH, associé à l'action de l'acide phytique, provoque la déstructuration de la membrane cellulaire, y compris la membrane nucléaire, la lyse des cellules végétales et provoque la dénaturation de l'ADN (les 2 brins de la double hélice sont séparés).

Le contrôle du pH montre que celui-ci reste basique et se stabilise entre 9 et 11 à la fin de l'étape c).

L'étape d) d'extraction dure préférentiellement au moins 1h, à une température comprise entre 40 et 80°C. De préférence l'étape dure 1 heure. Avantageusement l'étape est réalisée à une température comprise entre 60°C et 80°C. Encore plus préférentiellement, l'étape est réalisée à 80 °C. Pendant cette étape, le mélange est avantageusement mis sous agitation modérée.

Dans une étape e), on purifie le mélange obtenu en d) de manière à éliminer les feuilles de thés solides résiduelles et récupérer la partie soluble qui constitue l'extrait brut aqueux selon l'invention. Toute méthode connue par l'homme du métier pourra être utilisée pour réaliser ces étapes de purification. De préférence, le mélange est filtré directement sur des filtres de porosité supérieure ou égale à 30 µm de façon à récolter le filtrat. Le mélange obtenu en c) peut également être centrifugé à faible vitesse, par exemple pendant au moins 10 min à 4000 g, de manière à sédimenter la matière végétale résiduelle dans le culot et récupérer l'extrait brut aqueux dans le surnageant.

Dans une étape f), on ajuste le pH à une valeur comprise entre 6 et 8.

Dans une étape g) on ajoute au mélange du charbon actif en poudre. Préférentiellement on utilise un mélange de deux types de charbons différents, et encore plus préférentiellement 0,25 % (poids/poids) de chacun des deux types de charbons activés afin de créer un effet synergique sur la décoloration de l'extrait. Les charbons actifs sont sélectionnés pour leurs capacités à appauvrir l'extrait en polyphénols, ces derniers étant principalement responsables de la couleur de l'extrait. Cette étape dure préférentiellement trente minutes, à une température comprise entre 40 et 50°C sous agitation pour un résultat optimal.

Dans une étape h), le mélange obtenu à l'étape g) est purifié en éliminant les particules de charbon au moyen de l'utilisation de filtres de porosité supérieure ou égale à 30 µm de façon à obtenir un filtrat clarifié.

Dans une étape i), le filtrat obtenu à l'étape h) est mis en contact avec des polyvinylpolypyrrolidones (PVPP) en poudre afin de retirer un maximum de composés phénoliques, tels que des polyphénols de haut poids moléculaire, types tanins, contenus dans le mélange qui n'auraient pas été retenus par l'étape de traitement par les charbons et qui apportent de la couleur à l'extrait et risqueraient de perturber la stabilité de l'extrait final. De préférence, on utilise une concentration finale comprise entre 5 et 30 g/l de PVVP. Encore plus préférentiellement, on utilise une concentration finale de 10 g/l de PVPP.

Cette étape peut durer entre 10 et 30 min et préférentiellement dix minutes, à une température comprise entre 40 et 50°C sous agitation pour un résultat optimal.

Le procédé selon l'invention et en particulier les étapes g) à i) permettent d'obtenir des extraits qui contiennent peu, voire pas de polyphénols de type catéchine, épicatéchine ou encore épigallocatéchine. Seuls les extraits obtenus à partir de thé pu'er contiennent une faible concentration de catéchine. Des analyses par chromatographie sur couche mince ont montré qu'aucun des extraits de thé de l'invention ne contenaient d'épicatéchine ou d'épigallocatéchine.

A l'étape j) le mélange obtenu à l'étape i) est clarifié en éliminant les particules de PVPP par l'utilisation de filtres de porosité supérieure à 25 µm, suivie d'une filtration utilisant un filtre de 0,8 µm de porosité.

Dans une étape k), on ajuste ensuite le pH du mélange obtenu lors de l'étape j) à une valeur comprise entre 6 et 6,5. On obtient alors un extrait brut selon l'invention.

Le pH peut être ajusté par ajout d'une solution d'acide chlorhydrique (HCl) ou tout autre acide équivalent, compatible avec une utilisation cosmétique, tel que de l'acide citrique. L'étape d'ajustement du pH à l'étape k) du procédé selon l'invention est une étape indispensable pour obtenir un extrait stable sans précipitation des composés phytochimiques d'intérêt. En effet, ce pH est optimal pour le maintien en suspension des ARN de petit poids moléculaire, ainsi que des autres phytomolécules d'intérêt ; comme les sucres, les composés phénoliques, les acides organiques, la théine et la théanine.

En effet, un pH inférieur à 6 peut entraîner la précipitation des acides nucléiques en général, donc celle des ARN de petit poids moléculaire d'une longueur d'au maximum 150 nucléotides, ou encore la précipitation de certains sucres, polyphénols, et protéines. L'étape d'ajustement du pH à l'étape i) du procédé selon l'invention est une étape indispensable pour avoir une stabilité optimale de l'extrait.

L'extrait obtenu à l'étape k) peut être dilué avec un solvant physiologiquement acceptable pour un usage cosmétique afin de stabiliser le produit et d'augmenter sa préservation dans le temps. On obtient alors un extrait dilué.

L'extrait est dilué de sorte à obtenir un poids sec compris entre 4 et 20 g/kg, son pH est ajusté entre 5,8 et 6,5, préférentiellement entre 6,0 et 6,5. Cette étape permet d'améliorer la stabilité du produit dans le temps.

L'invention a pour deuxième objet des extraits aqueux de feuilles de thé obtenus à partir de thé frais, de thé pu'er, de thé matcha ou de thé vert, enrichis en petits ARN d'une longueur d'au maximum 150 nucléotides, en sucres, en composés phénoliques, en acides organiques, en théine et en théanine et dépourvus d'ADN, susceptibles d'être obtenus par le procédé décrit ci-dessus. Cet extrait ne contient pas d'ADN (acide désoxyribonucléique).

L'invention a encore pour objet des extraits aqueux de feuilles de thé obtenus à partir de thé frais, de thé pu'er, de thé matcha ou de thé vert, enrichis en petits ARN d'une longueur d'au maximum 150 nucléotides, en sucres, en composés phénoliques, en acides organiques, en théine, en théanine directement obtenus par le procédé décrit ci-dessus. Cet extrait ne contient pas d'ADN (acide désoxyribonucléique).

En utilisant le procédé de l'invention selon les étapes a) à k) on obtient des extraits bruts concentrés aqueux de thé de couleur ambre à ambre foncé ayant un poids sec de 5 à 30 g/kg, contenant 0,5 à 10 g/kg de sucres, 0,050 à 2 g/kg d'acides organiques, 0,050 à 5 g/kg de composés phénoliques dont 0 à 200 mg/kg de catéchine, 0,020 à 2 g/kg de théine et 0,020 à 2 g/kg de théanine et 50 à 350 mg/kg d'ARN de petit poids moléculaire d'une longueur d'au maximum 150 nucléotides. Néanmoins, les feuilles de thé étant soumises à des traitements variés, les extraits obtenus peuvent présenter une variabilité importante en fonction de facteurs tels que le lieu ou l'année de récolte, la saison, les conditions climatiques, le stress biotique, etc.

L'extrait ainsi obtenu peut ensuite être dilué dans un solvant physiologiquement acceptable pour un usage cosmétique, de telle sorte que la concentration de l'extrait est alors ajustée à un poids sec compris entre 4 et 20 g/kg.

A titre de solvants physiologiquement acceptables, on peut citer l'eau, le glycérol, l'éthanol, le propanediol, le butylène glycol, ainsi que leur version naturelle, le dipropylène glycol, les diglycols éthoxylés ou propoxylés, les polyols cycliques ou tout mélange de ces solvants. L'éthanol, le butylène glycol, le propanediol et la glycérine existent sous forme de solvants synthétiques ou dérivés de plantes, cette dernière forme étant qualifiée de version naturelle.

Ainsi l'extrait obtenu peut ainsi être mélangé avec 30 à 50 % de butylène glycol dérivé de plante, ou 30 à 50 % de propanediol dérivé de plante ou encore 30 à 70 % de glycérine dérivée de plante.

Préférentiellement, l'extrait obtenu par le procédé selon l'invention est dilué dans du butylène glycol de telle sorte que l'extrait dilué comprenne une concentration finale en butylène glycol de 30 % ou de 50%.

Cet extrait dit dilué a un poids sec compris entre 4 à 20 g/kg, une concentration de 0,2 à 5 g/kg de sucres, 0,030 à 1 g/kg d'acides organiques, 0,030 à 3 g/kg de composés phénoliques dont 0 à 120 mg/kg de catéchine, 0,010 à 1 g/kg de théine et 0,010 à 1 g/kg de théanine et 10 à 250 mg/kg d'ARN de petit poids moléculaire d'une longueur d'au maximum 150 nucléotides.

A titre d'exemple non limitatif, on peut citer un extrait dilué de thé vert contenant plus particulièrement des sucres à une concentration de 1,4 g/kg, des acides organiques à une teneur de 95.5 mg/kg, 32 mg/kg de composés phénoliques et 77 mg/kg d'ARN de petits poids moléculaires d'une longueur d'au maximum 150 nucléotides.

L'extrait de l'invention comprend ainsi une large gamme de phytomolécules pouvant présenter des effets bénéfiques sur la peau, sans présenter de risque d'irritation cutanée ou autre dommage pour la santé.

Par exemple, les sucres participent activement à l'hydratation des couches supérieures de l'épiderme, et ce faisant à la résistance aux agressions extérieures, sans présenter aucun effet indésirable. Les extraits de thé de l'invention contiennent plus particulièrement des mono- et des polysaccharides.

*Camellia sinensis* fait partie de la famille des Théaceae qui produit de la théine, un puissant insecticide naturel. La théine est aussi appelée caféine ou 1,3,7-triméthylxanthine ou méthylthéobromine. Au-delà de ses effets reconnus sur le système nerveux et cardio-vasculaire, la théine a également des effets bénéfiques sur la peau en particulier sur le relâchement cutané.

La plante produit également de la théanine, un acide aminé non protéique communément présent dans les feuilles de thé. La théanine est reconnue pour son activité relaxante sur le cerveau permettant la réduction du stress mental et physique.

Les extraits de thé selon l'invention contiennent également des composés phénoliques, tels que les polyphénols, les acides phénoliques, les flavonoïdes comme la catéchine, cependant cette famille de molécules a été partiellement ôtée par des étapes de traitement par du charbon et du PVPP. Cet appauvrissement relatif en composés phénoliques permet d'augmenter la stabilité du produit dans le temps et d'obtenir un extrait non phototoxique.

Dans un mode de réalisation particulier de l'invention l'extrait aqueux dilué selon l'invention contient au maximum 1,5 g/kg de composés phénoliques dont au maximum 120 mg /kg de catéchine.

Dans un autre mode de réalisation particulier de l'invention l'extrait aqueux dilué selon l'invention contient au maximum 1,2 g/kg de composés phénoliques dont au maximum 30 mg /kg de catéchine.

Dans un autre mode de réalisation particulier de l'invention l'extrait aqueux dilué selon l'invention contient au maximum 100 mg/kg de composés phénoliques et ne contient pas de catéchine.

Dans encore un autre mode de réalisation particulier de l'invention l'extrait aqueux dilué selon l'invention contient au maximum 50 mg/kg de composés phénoliques et ne contient pas de catéchine.

Dans un mode de réalisation particulier de l'invention l'extrait aqueux dilué selon l'invention contient au maximum 3 g/kg de composés phénoliques et ne contient pas de catéchine.

Un troisième objet de l'invention est une composition cosmétique comprenant une quantité efficace d'au moins un extrait aqueux de thé choisi parmi le thé frais, le thé pu'er, le thé matcha, le thé vert, ou tout mélange de ceux-ci, obtenus selon l'invention, en tant qu'ingrédient actif, et un milieu physiologiquement acceptable.

Avantageusement, les extraits de thé selon l'invention sont ajoutés dans un milieu physiologiquement acceptable à une concentration de 0,05 à 5 % en poids par rapport au poids total de la composition, préférentiellement à une concentration de 0,1 à 2,5 % en poids par rapport au poids total de la composition.

La composition utilisable selon l'invention est formulée pour être appliquée par toute voie appropriée, notamment orale, ou topique externe, et la formulation des compositions sera adaptée par l'homme du métier.

Préférentiellement, les compositions selon l'invention se présentent sous une forme adaptée à l'application par voie topique. Ces compositions doivent donc contenir un milieu physiologiquement acceptable, c'est-à-dire compatible avec la peau et les phanères, sans risque d'inconfort lors de leur application et couvrent toutes les formes cosmétiques adaptées.

Les compositions pour la mise en œuvre de l'invention pourront notamment se présenter sous forme d'une solution ou d'un gel aqueux, hydroalcooliques ou huileux, d'une émulsion huile-dans-eau, eau-dans-huile ou émulsions multiples ; elles peuvent aussi se présenter sous forme de suspensions, ou encore poudres, adaptées à une application sur la peau, les muqueuses, les lèvres et/ou les cheveux.

Ces compositions peuvent être plus ou moins visqueuses et avoir également l'aspect d'une crème, d'une lotion, d'un fluide, d'un lait, d'un sérum, d'une pommade, d'un gel, d'une pâte, d'un baume ou d'une mousse. Elles peuvent aussi se présenter sous forme solide, comme un stick ou être appliquées sur la peau sous forme d'aérosol.

A titre de milieu physiologiquement acceptable communément utilisé dans le domaine d'application envisagé, on peut citer par exemple des adjuvants nécessaires à la formulation, tels que des solvants, des épaississants, des gélifiants, des diluants, des émulsionnants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, des esters, des huiles ou beurres végétaux, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre chacun à 0,01 à 20 % du poids total de la composition. Lorsque la composition selon l'invention est une émulsion, la phase grasse peut représenter de 2 à 90 % en poids et de préférence de 5 à 30 % en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30 % en poids par rapport au poids total de la composition.

Selon un autre mode de réalisation avantageux de l'invention, les extraits aqueux de thé de l'invention peuvent être encapsulés ou inclus dans un vecteur cosmétique tels que les liposomes ou toute autre nano capsule ou microcapsule utilisées dans le domaine de la cosmétique ou adsorbés sur des polymères organiques poudreux, des supports minéraux comme les talcs et bentonites.

Avantageusement, la composition selon l'invention peut comprendre, outre l'ingrédient actif selon l'invention, au moins un autre agent actif présentant des effets cosmétiques similaires et/ou complémentaires à ceux de l'invention.

Par exemple, le ou les agents actifs additionnels peuvent être choisis parmi : les agents anti-âge, raffermissants, éclaircissants, hydratants, drainants, favorisant la microcirculation, exfoliants, desquamants, stimulant la matrice extracellulaire, activant le métabolisme énergétique, antibactériens, antifongiques, apaisants, anti-radicalaires, anti-UV, anti-acné, anti-inflammatoires, anesthésiques, procurant une sensation de chaleur, procurant une sensation de fraîcheur, amincissants.

De tels agents actifs additionnels peuvent être choisis dans les groupes comprenant :
- la vitamine A et notamment l'acide rétinoïque, le rétinol, le rétinol propionate, le rétinol palmitate ;
- la vitamine B3 et plus particulièrement le niacinamide, le nicotinate de tocophérol ;
- la vitamine B5, la vitamine B6, la vitamine B12, le panthénol ;
- la vitamine C, notamment l'acide ascorbique, l'ascorbyl glucoside, l'ascorbyl tétrapalmitate, magnésium et sodium ascorbyl phosphate ;
- les vitamines E, F, H, K, PP, le coenzyme Q10 ;
- les inhibiteurs de métalloprotéinase, ou un activateur des TIMP ;
- la DHEA, ses précurseurs et dérivés ;
- les acides aminés tels que l'arginine, ornithine, hydroxyproline, hydroxyproline dipalmitate, palmitoylglycine, hydroxylysine, méthionine et ses dérivés, composés acides aminés N-acylés ;
- les peptides naturels ou de synthèse, incluant les di-, tri-, tetra-, penta- et hexapeptides et leurs dérivés lipophiles, isomères et complexés avec d'autres espèces telles qu'un ion métal (par exemple cuivre, zinc, manganèse, magnésium, et autres). A titre d'exemples, on peut citer les peptides commercialement connus sous les noms de MATRIXYL^{®}, ARGlRELlNE^{®}, CHRONOGEN^{™}, LAMINIXYL IS^{™}, PEPTIDE Q10^{™}, COLLAXYL^{™} (brevet FR2827170, ASHLAND^{®}), PEPTIDE VINCI 01^{™} (brevet FR2837098, ASHLAND^{®}), PEPTIDE VINCI 02^{™} (brevet FR2841781, ASHLAND^{®}), ATPeptide^{™} (brevet FR2846883, ASHLAND^{®}) ou encore le peptide de synthèse de séquence Arg-Gly-Ser-NH2, commercialisé sous le nom ATPeptide^{™} par ASHLAND^{®} ;
- l'extrait d'Artémia salina, commercialisé sous le nom GP4G^{™} (FR2817748, ASHLAND^{®}) ;
- les extraits peptidiques végétaux tels que les extraits de lin (Lipigénine^{™}, brevet FR2956818, ASHLAND^{®}), les extraits de soja, d'épeautre, de vigne, de colza, de lin, de riz, de maïs, de pois ;
- les extraits de levures, par exemple le Dynagen^{™}, (brevet FR2951946, ASHLAND^{®}) ou l'Actopontine^{™} (brevet FR2944526, ASHLAND^{®}) ;
- l'acide déhydroacétique (DHA) ;
- les phystostérols d'origine synthétique ou naturelle ;
- l'acide salicylique et ses dérivés, les alpha- et bêta-hydroxyacides, les silanols ;
- les sucres aminés, glucosamine, D-glucosamine, N-acetyl glucosamine, N-acetyl-D-glucosamine, mannosamine, N-acetyl mannosamine, galactosamine, N-acetyl galactosamine
- les extraits de polyphénols, isoflavones, flavonoïdes, tels que les extraits de raisin, les extraits de pin, les extraits d'olive ;
- les lipides tels que les céramides ou les phospholipides, les huiles d'origine animale, telles que le squalène ou le squalane ; les huiles végétales, telles que l'huile d'amande douce, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, les beurres de karité, de cacao, de babassu ;
- tous écrans UV et filtres solaires ;
- l'AMP cyclique et ses dérivés, les agents activateurs de l'enzyme adénylate cyclase et les agents inhibiteurs de l'enzyme phosphodiestérase, l'extrait de Centella asiatica, l'asiaticoside et l'acide asiatique, les méthyls xanthines, la théophylline, la théobromine, la forskoline, l'esculine et l'esculoside, les inhibiteurs d'ACE, le peptide Val-Trp, l'inhibiteur du neuropeptide Y, l'enkephaline, l'extrait de Ginkgo biloba, l'extrait de dioscorea, la rutine, l'extrait de yerba mate, l'extrait de guarana, les oligosaccharides, les polysaccharides, la carnitine, l'extrait de lierre, l'extrait de fucus, l'extrait hydrolysé de Prunella vulgaris, l'extrait hydrolysé de Celosia cristata, l'extrait d'Anogeissus leiocarpus, l'extrait de feuilles de Manihot utilissima, la palmitoylcarnitine, la carnosine, la taurine, l'extrait de sureau, les extraits d'algues tel que l'extrait de Palmaria Palmata ;
- l'acide hyaluronique ou sodium hyaluronate ainsi que toutes ses fractions et promoteurs ;
- le menthol et menthyl lactate et autres actifs à effet frais ;
- l'aloe vera, le bisabolol, l'allantoin et autres actifs à effet apaisant ;
- le Vanillyl Butyl Ether et autres actifs à effet chauffant ;
- le Benzoyl Peroxide et autres actifs anti-acné.

L'invention a pour quatrième objet l'utilisation cosmétique d'une composition comprenant les extraits de thé de l'invention, provenant de thé frais, de thé pu'er, de thé matcha ou de thé vert, ou tout mélange de ceux-ci, pour le soin de la peau, du cuir chevelu et des phanères, plus particulièrement pour protéger la peau des agressions externes et de l'oxydation, lutter contre les signes du vieillissement cutané, augmenter la photoprotection, éclaircir la peau, améliorer l'hydratation de la peau.

Dans un mode de réalisation particulier, l'invention a encore pour objet l'utilisation cosmétique d'une composition comprenant les extraits de thé de l'invention obtenus à partir de thé frais, de thé matcha ou de thé vert, ou tout mélange de ceux-ci, pour le soin de la peau, du cuir chevelu et des phanères, plus particulièrement pour protéger la peau des agressions externes et de l'oxydation, lutter contre les signes du vieillissement cutané, augmenter la photoprotection, éclaircir la peau, améliorer l'hydratation de la peau.

La peau est un organe composé de plusieurs couches (derme, épiderme et stratum corneum), qui recouvre toute la surface du corps et assure des fonctions protectrices vis-à-vis des agressions externes, sensitives, immunitaires, métaboliques, thermorégulatrices ou encore de barrière limitant la déshydratation.

En particulier, le stratum corneum assure un rôle de barrière physique protectrice, communément nommé « fonction barrière de la peau ». Cette fonction revêt une importance majeure dans l'homéostasie tissulaire et dans la protection vis-à-vis de l'environnement extérieur.

L'aspect de la peau peut être modifié par des altérations internes (vieillissement intrinsèque, maladies et changements hormonaux tels que la grossesse) ou externes (facteurs environnementaux, tels que la pollution, la lumière du soleil, les pathogènes, les variations de température, etc.). Toutes ces altérations affectent non seulement la peau, mais aussi les annexes kératiniques telles que les poils, les cils, les sourcils, les ongles et les cheveux.

### Exemples

A titre illustratif, des exemples de mode de réalisation du procédé selon l'invention sont décrits ci-dessous.

### Exemple 1 : Préparation d'extraits de thé frais, de thé pu'er, de thé matcha ou de thé vert (Camellia sinensis), enrichis en petits ARN selon le procédé de l'invention :

Dans une première étape a) pour fabriquer l'extrait, ont été utilisés du thé matcha (thé vert japonais sous forme de poudre), du thé pu'er (thé origine du Yunnan sous forme de feuilles entières compressées), du thé vert (thé sous forme de feuilles séchées originaire de Chine) et du thé frais (feuilles directement collectées d'un arbre) tous issus de l'espèce *Camellia sinensis.*

Le thé vert ou le thé pu'er est pesé pour représenter 3 % de matière première engagée dans le procédé, soit 30 g pour 1 kg. La quantité d'eau distillée ajoutée est de 968 g.

Le thé frais est pesé pour représenter 6 % de matière première engagée, soit 60 g pour 1 kg du fait de la teneur en eau représentant la moitié du poids frais des feuilles. La quantité d'eau distillée ajoutée est de 938 g.

Le mélange feuilles de thé et eau est broyé dans un mixeur pour permettre une meilleure extraction des phytocomposés grâce à une plus grande surface d'échange entre la matière solide, le thé, et la solution aqueuse d'extraction.

Le thé matcha, dont les feuilles séchées sont déjà en poudre sont ajoutées directement à un mélange de 968 g d'eau distillée et de 2 g/l soit 3 mM d'acide phytique homogénéisés au préalable.

Dans une étape b), de l'acide phytique à 2 g/l (soit 3 mM final) est ajouté aux mélanges eau et feuilles de thé broyées (sauf pour le thé matcha).

Dans une étape c), le pH est ajusté à 10,5 pour que l'extraction soit optimale et permettre un enrichissement de l'extrait en ARN de petits poids moléculaires ainsi qu'en diverses phytomolécules.

Dans une étape d) le mélange est chauffé 1 h à 80°C sous agitation modérée.

Dans une étape e) le mélange est filtré à l'aide de filtres de porosité de 30 µm, pour séparer la matière solide du filtrat. Trois filtrations séquentielles sont ensuite réalisées à l'aide de filtres de porosité décroissante afin de clarifier l'extrait végétal jusqu'à une filtration à 3 µm de porosité.

Etape f) Le pH du filtrat récolté à l'étape e) supérieur à 9 en moyenne est ajusté entre 6 et 8 pour se placer à un pH optimal pour le traitement aux charbons actifs qui suit.

Etape g) Un traitement par un duo de charbons actifs est effectué afin de décolorer l'extrait en ôtant notamment des polyphénols. 2,5 g de chaque type de charbon par litre d'extrait sont ajoutés. La durée de traitement est de trente minutes à une température comprise entre 40 et 50°C.

Etape h) : Les charbons sont éliminés en passant l'extrait sur des filtres de porosité de 30 µm.

A l'étape i) un second traitement de décoloration du filtrat obtenu en g) est effectué en ajoutant du polyvinylpolypyrrolidone (PVPP) en poudre. 10 g/l de PVPP sont ajoutés à l'extrait afin de retirer une partie des tanins contenus dans l'extrait. La durée de traitement est de 10 minutes à une température comprise entre 40 et 50°C.

A l'étape j) des filtrations séquentielles sur filtres de porosité décroissante sont alors réalisées afin de retirer le PVPP de l'extrait végétal, une première filtration de 30 µm permet de retenir le PVPP suivi de filtrations successives allant jusqu'à une porosité de 0,8 µm afin de clarifier l'extrait.

A l'étape k) le pH est contrôlé, puis ajusté à une valeur comprise entre 6 et 6,5 avec une solution d'acide citrique ou d'acide chlorhydrique.

On obtient des extraits bruts aqueux ayant des poids secs compris entre 5 et 30 g/kg.

L'extrait obtenu à l'étape k) est dilué avec du butylène glycol à hauteur de 30 %. Ce solvant physiologiquement acceptable pour un usage cosmétique permet de stabiliser le produit et d'augmenter sa préservation dans le temps. On obtient alors un extrait dilué dont on ajuste le pH entre 6,0 et 6,5.

L'extrait dilué ainsi obtenu possède un poids sec compris entre 4 et 20 g/kg.

Les extraits de thé ainsi obtenus selon le procédé décrit dans cet exemple sont appelés PSR (Plant small RNA) thé dans les tableaux ou figures présentés dans l'invention.

### Exemple 2 : Préparation d'extraits de thé (Camellia sinensis) par une méthode d'extraction dite conventionnelle :

Dans un but de comparaison, des extraits de thé obtenus par une extraction dite conventionnelle ou classique ont été réalisés. Ce procédé d'extraction a été choisi car il est optimal pour extraire les différents types de composés phénoliques et autres molécules polaires comme les sucres, les acides aminés ce qui en fait un bon procédé de référence pour réaliser des comparaisons avec le procédé de l'invention appliqué dans l'exemple 1.

Dans une première étape pour fabriquer 1 kg d'extrait, les mêmes thés que dans l'exemple 1 ont été utilisés ; thé matcha (thé vert japonais sous forme de poudre), thé pu'er (thé origine du Yunnan sous forme de feuilles entières compressées), thé vert (thé sous forme de feuilles séchées originaire de Chine) et thé frais (feuilles directement collectées d'un arbre) tous issus de l'espèce *Camellia sinensis.*

Les feuilles de thés séchées ou réduites en poudre comme le thé matcha, thé pu'er et thé vert sont pesées pour représenter 3 % de matière première engagée dans le procédé soit 30 g pour 1 kg auquel on mélange 970 g d'eau distillée.

Les feuilles de thé frais qui représentent 6 % de matière première engagée soit 60 g pour 1 kg du fait de leur teneur naturelle en eau sont mélangées à 940 g d'eau distillée. Une étape de broyage a été appliquée pour le thé frais, le thé vert et le thé pu'er. Le pH d'extraction n'est pas ajusté et se situe entre 5 et 7.

Le mélange est ensuite chauffé 1 h à 45°C sous agitation.

Le mélange est ensuite filtré à l'aide de filtres de porosité de 30 µm, pour séparer la matière solide du filtrat. Des filtrations séquentielles sur filtres de porosité décroissante sont alors réalisées afin de clarifier l'extrait végétal jusqu'à une filtration à 1 µm de porosité.

A l'issue de cette étape, un traitement au charbon est effectué afin de décolorer l'extrait en ôtant notamment des polyphénols. 2,5 g de chaque charbon par litre d'extrait est ajouté. La durée de traitement est de trente minutes à une température comprise entre 40 et 50°C et à un pH compris entre 6 et 8. Une filtration de 30 µm est réalisée pour ôter les charbons de l'extrait.

Puis un second traitement de décoloration est effectué en ajoutant du polyvinylpolypyrrolidone (PVPP) en poudre. 10 g/l est ajouté à l'extrait afin de retirer une partie des tanins contenus dans l'extrait. La durée de traitement est de 10 minutes à une température comprise entre 40 et 50°C. Des filtrations séquentielles sur filtres de porosité décroissante sont alors réalisées afin de clarifier l'extrait végétal jusqu'à une filtration à 0,2 µm.

Le pH est contrôlé, puis il est ajusté entre 6 et 6,5 avec une solution d'acide citrique ou d'acide chlorhydrique ou de soude.

On obtient des extraits bruts aqueux ayant des poids secs compris entre 1 et 5 g/kg. Le procédé mis en œuvre dans cet exemple 2 a pour but de préparer un extrait contrôle pour obtenir des données analytiques comparatives par rapport aux différents extraits de thé obtenus par le procédé de l'invention. Les résultats obtenus sont illustrés dans les figures et dans le texte de la demande.

### Exemple 3 : Caractéristiques des extraits de thé obtenus selon l'exemple 1

L'extrait brut de thé frais réalisé à partir de feuilles fraîches directement récoltées sur l'arbre a un poids sec de 6,5 g/kg. L'analyse physico-chimique montre que l'extrait obtenu présente une concentration de 2 g/kg de sucres totaux, 120 g/kg d'acides organiques totaux, 31 mg/kg de composés phénoliques totaux et 120 mg/kg d'ARN de petits poids moléculaires d'une longueur d'au maximum 150 nucléotides.

L'extrait est ensuite dilué avec un solvant cosmétique physiologiquement acceptable et permettant de garantir une meilleure stabilité et une meilleure conservation de l'extrait dans le temps, la quantité de solvant peut aller de 30 à 70 %.

La dilution est réalisée avec du butylène glycol dérivé de plante de manière à obtenir une concentration finale de 30 % de butylène glycol et 70 % d'extrait de thé. L'extrait ainsi dilué possède alors un poids sec de 4,3 g/kg, et présente une concentration de 1,4 g/kg de sucres totaux, 83 mg/kg d'acides organiques totaux, 48 mg/kg de composés phénoliques totaux et 74 mg/kg d'ARN de petits poids moléculaires d'une longueur d'au maximum 150 nucléotides.

L'extrait brut de thé vert réalisé à partir de feuilles séchées originaires de Chine a un poids sec de 10,2 g/kg. L'analyse physico-chimique montre que l'extrait obtenu présente une concentration de 1,5 g/kg de sucres totaux, 137 mg/kg d'acides organiques totaux, 1,3 g/kg de composés phénoliques totaux et 110 mg/kg d'ARN de petits poids moléculaires d'une longueur d'au maximum 150 nucléotides.

L'extrait est ensuite dilué avec un solvant cosmétique physiologiquement acceptable et permettant de garantir une meilleure stabilité et une meilleure conservation de l'extrait dans le temps, la quantité de solvant peut aller de 30 à 70 %.

La dilution est réalisée avec du butylène glycol dérivé de plante de manière à obtenir une concentration finale de 30 % de butylène glycol et 70 % d'extrait de thé. L'extrait ainsi dilué possède alors un poids sec de 7,1 g/kg, et présente une concentration de 1,1 g/kg de sucres totaux, 96 mg/kg d'acides organiques totaux, 931 mg/kg de composés phénoliques totaux et 77 mg/kg d'ARN de petits poids moléculaires d'une longueur d'au maximum 150 nucléotides.

L'extrait brut de thé matcha préparé à partir de feuilles broyées originaire du Japon a un poids sec de 14,2 g/kg. L'analyse physico-chimique montre que l'extrait obtenu présente une concentration de 2,4 g/kg de sucres totaux, 179 mg/kg d'acides organiques totaux, 2 g/kg de composés phénoliques totaux et 87 mg/kg d'ARN de petits poids moléculaires d'une longueur d'au maximum 150 nucléotides.

L'extrait est ensuite dilué avec un solvant cosmétique physiologiquement acceptable et permettant de garantir une meilleure stabilité et une meilleure conservation de l'extrait dans le temps, la quantité de solvant peut aller de 30 à 70 %.

La dilution est réalisée avec du butylène glycol dérivé de plante de manière à obtenir une concentration finale de 30 % de butylène glycol et 70 % d'extrait de thé. L'extrait ainsi dilué possède alors un poids sec de 10,2 g/kg, et présente une concentration de 1,7 g/kg de sucres totaux, 125 mg/kg d'acides organiques totaux, 1420 mg/kg de composés phénoliques totaux et 61 mg/kg d'ARN de petits poids moléculaires d'une longueur d'au maximum 150 nucléotides.

L'extrait brut de thé pu'er réalisé à partir de feuilles entières compressées originaires du Yunnan a un poids sec de 13,8 g/kg. L'analyse physico-chimique montre que l'extrait obtenu présente une concentration de 3,3 g/kg de sucres totaux, 137 mg/kg d'acides organiques totaux, 193 mg/kg de composés phénoliques totaux et 250 mg/kg d'ARN de petits poids moléculaires d'une longueur d'au maximum 150 nucléotides.

L'extrait est ensuite dilué avec un solvant cosmétique physiologiquement acceptable et permettant de garantir une meilleure stabilité et une meilleure conservation de l'extrait dans le temps, la quantité de solvant peut aller de 30 à 70 %.

La dilution est réalisée avec du butylène glycol dérivé de plante de manière à obtenir une concentration finale de 30 % de butylène glycol et 70 % d'extrait de thé. L'extrait ainsi dilué possède alors un poids sec de 8 g/kg, et présente une concentration de 1,1 g/kg de sucres totaux, 137 mg/kg d'acides organiques totaux, 1176 mg/kg de composés phénoliques totaux et 177 mg/kg d'ARN de petits poids moléculaires d'une longueur d'au maximum 150 nucléotides.

### Exemple 4 : Quantification des différents composants des extraits dilués de thé obtenus dans les exemples 1 et 2

Le poids sec de chaque type d'extrait, obtenu selon le procédé d'invention ou selon un procédé conventionnel a été mesuré. Le poids sec représente le poids de l'extrait après que l'eau et les composés volatiles se soient évaporés après 12 h d'étuvage à 105°C.

Les extraits secs des différents extraits de thé obtenus par le procédé selon l'invention ont un poids sec supérieur à celui des extraits de thé obtenus par l'extraction conventionnelle. Le rendement d'extraction est donc supérieur quand le procédé de l'exemple 1 est appliqué pour extraire les différents types de thé, voir tableau 1.

**[Tableau 1]**

| | Extrait PSR thé frais | Extrait thé frais convent ionnel | Extrait PSR thé vert | Extrait thé vert convent ionnel | Extrait PSR thé matcha | Extrait thé matcha convent ionnel | Extrait PSR thé pu'er | Extrait thé pu'er convent ionnel |
|---|---|---|---|---|---|---|---|---|
| Poids sec (g/kg) | 4,5 | 1 | 7,1 | 2,3 | 10 | 3,1 | 8 | 3,6 |
| Rendem ent d'extracti on (%) | 15 | 3,3 | 23,7 | 7,7 | 33,3 | 10,3 | 46 | 24 |

La teneur totale en sucres dans les différents extraits de thé obtenus par le procédé de l'invention versus les extraits obtenus par le procédé conventionnel a été déterminée par dosage spectrophotométrique issu d'une adaptation du dosage décrit par Dubois et al. (1956) (Dubois et al., "Méthode colorimétrique pour la détermination des sucres et des substances apparentées", Anal. Chem., 1956, 28 (3), 350-356). Cette analyse consiste en la dissolution de la matière première dans l'acide sulfurique concentré puis en réagissant avec du phénol pour former un complexe coloré. L'absorbance du complexe est lue sur le spectrophotomètre à 490 nm. La teneur en sucre est déterminée à l'aide d'une courbe standard de glucose.

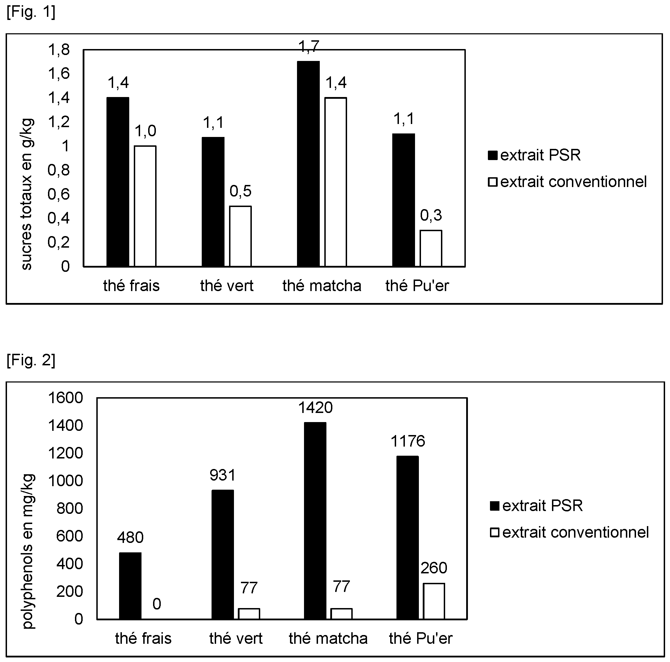

L'analyse met en évidence la plus forte concentration de sucres extraits grâce au procédé décrit dans l'invention pour tous les types de thé, frais, vert, marcha, thé pu'er comparativement avec un procédé d'extraction conventionnel, voir [fig. 1].

La teneur totale en polyphénols des extraits de thés selon l'invention versus les extraits de thés conventionnels a été déterminée par la méthode de dosage spectrophotométrique de Folin-Ciocalteu (Singleton et al., Analyse des phénols totaux et d'autres substrats d'oxydation et antioxydants au moyen du réactif Folin-Ciocalteu, 1999, 299: 152). Les composés de type polyphénols présents dans l'échantillon réagissent avec le réactif de Folin-Ciocalteu, l'oxydation du réactif donne une couleur bleue. L'absorbance de l'échantillon est lue sur le spectrophotomètre à 760 nm. La concentration totale en polyphénol est exprimée en équivalents d'acide gallique à l'aide d'une courbe standard d'acide gallique. L'analyse met en évidence la plus forte concentration de polyphénol extraits grâce au procédé décrit dans l'invention pour tous les types de thé, frais, vert, matcha et le thé pu'er comparativement avec un procédé d'extraction conventionnel, voir [fig. 2].

La caractérisation et la quantification des acides organiques contenus dans les extraits de thés selon l'invention versus les extraits de thés conventionnels a été réalisée. Une analyse chromatographique liquide haute performance, couplée à un détecteur de masse a été utilisée. Tous les échantillons ont été séparés sur une colonne EC 150 / 4,6 Nucleoshell RP 18plus-5µm (150x4,6 mm) (Macherey Nagel: 763236.46) par un système HPLC Agilent 1260 (Agilent Technologies). Le débit était de 0,3 ml/min. La phase mobile consistait en une solution d'acide formique (HCOOH) à 0,01 % (A) et d'acétonitrile (B). Le programme de gradient a facilité l'élution tel que décrit dans le tableau 2.

[

**Tableau 2]**

| Temps (min) | A Eau/0,01 % HCOOH (%) | B Acetonitrile (ACN) (%) |
|---|---|---|
| 0 | 100 | 0 |
| 9 | 100 | 0 |
| 13 | 5 | 95 |
| 14 | 5 | 95 |
| 15 | 100 | 0 |
| 20 | 100 | 0 |

La température de la colonne a été maintenue à 25°C et le volume d'injection était de 5 µL. La détection a été effectuée par un détecteur de spectromètre de masse ACQUITY Qda (WATERS) avec une source d'ions électrospray en mode négatif. La source a été réglée à une tension capillaire de 0,8 kV et à une température de sonde de 600°C. M/z et la tension de cône ont été ciblés pour chaque composé tel que décrit dans le tableau 3.

**[Tableau 3]**

| Composés | Masse (m/z) | Cone voltage (V) |
|---|---|---|
| Acide tartrique | 149,0 | 10 |
| Acide malique | 132,9 | 3 |
| Acide citrique | 191,0 | 10 |
| Acide lactique | 88,9 | 5 |
| Acide succinique | 116,95 | 15 |
| Acide uronique | 193 | 10 |

L'identification des acides organiques a été réalisée en comparant les temps de rétention et les pics spectraux de masse de l'échantillon avec un étalon. L'estimation quantitative des acides organiques a été réalisée sur la base de l'aire des pics chromatographiques comparée aux aires des étalons.

L'analyse HPLC-MS qui permet de quantifier et d'identifier les acides organiques contenus dans les différents extraits de thé obtenus selon les exemples 1 et 2, montre que les extraits PSR de thé et les extraits conventionnels contiennent différents types d'acides organiques, principalement de l'acide citrique, lactique, malique et succinique, tel qu'illustré dans le tableau 4. L'acide tartrique et les acides uroniques n'ont pas été détectés ni dans les extraits PSR thé ni dans les extraits conventionnels.

**[Tableau 4]**

| Type d'extraits | Acide citrique | Acide lactique | Acide succinique | Acide malique | Acide tartrique | Acides uroniques |
|---|---|---|---|---|---|---|
| | mg/kg | | | | | |
| PSR thé frais | 39,0 | 7,7 | <LQ | 36,7 | nd | nd |
| thé frais conventionnel | 10,2 | 4,4 | nd | nd | nd | nd |
| PSR thé vert | 35,6 | 20,1 | 3,4 | 36,4 | nd | nd |
| thé vert conventionnel | 29,5 | 2,9 | 2,2 | 33,5 | nd | nd |
| PSR Matcha thé | 65,8 | 9,0 | 4,2 | 46,2 | nd | nd |
| thé matcha conventionnel | 62,3 | 6,1 | 6,6 | 35,7 | nd | nd |
| PSR thé pu'er | 77,1 | 8,1 | 5,1 | 46,5 | nd | nd |
| thé pu'er conventionnel | 66,8 | nd | 4,5 | 38,7 | nd | nd |
| LQ : limite de quantification ; nd : non détecté | | | | | | |

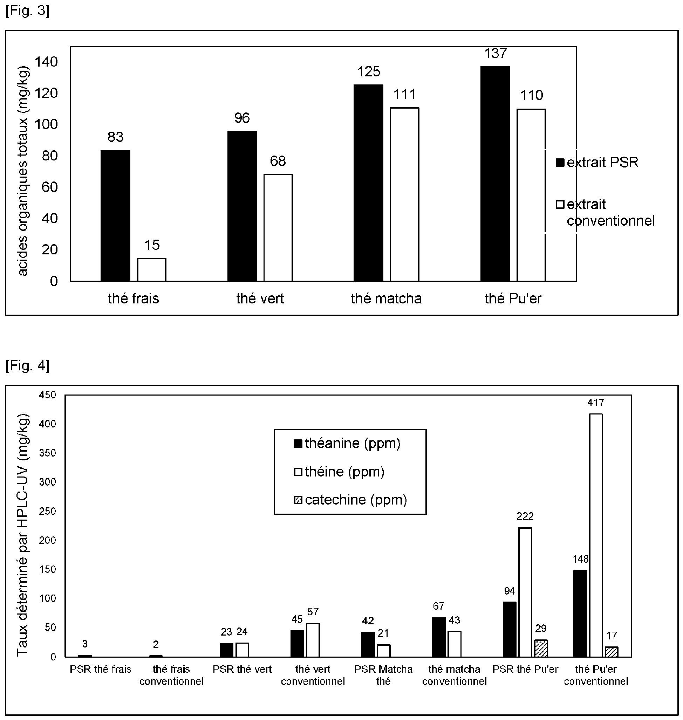

L'analyse montre aussi que ces acides organiques sont davantage extraits par le procédé de l'invention que par l'extraction conventionnelle tels que présentés sur la [fig. 3].

La caractérisation de la théanine contenue dans les extraits de thés selon l'invention versus les extraits de thés conventionnels a été déterminée. Les feuilles de thé contiennent cet acide aminé unique, qui représente 50 % de la quantité totale d'acides aminés chez le thé. Il s'agit d'un acide aminé non protéique. Une analyse chromatographique liquide haute performance, couplée à un détecteur UV a été utilisée. Tous les échantillons ont été séparés par une colonne Uptisphère C18-2 250 mm x 4,6 mm x 5 um (Interchim) par un système HPLC Agilent 1200 (Agilent Technologies). Le débit était de 0,4 ml/min. La phase mobile était constituée de 0,1 % d'acide phosphorique (H3PO4) en solution aqueuse (A) et d'acétonitrile (ACN) (B). L'élution a été facilitée par le programme de gradient comme suit, voir tableau 5 :

**[Tableau 5]**

| Temps (min) | A Eau/0,1 % H3PO4 (%) | B ACN (%) |
|---|---|---|
| 0 | 100 | 0 |
| 8 | 100 | 0 |
| 10 | 50 | 95 |
| 15 | 5 | 95 |
| 16 | 100 | 0 |

La température de la colonne a été maintenue à 30°C. Le volume d'injection était de 5 µL et la longueur d'onde de détection a été réglée à 196 nm et 210 nm en utilisant un détecteur UV. L'étalon de théanine a été acheté chez Sigma-Aldrich. L'identification de la théanine a été réalisée par comparaison du temps de rétention et du pic spectral UV de l'échantillon avec un standard. Une estimation quantitative de la théanine a été effectuée sur la base de l'aire des pics chromatographiques comparée à l'air de l'étalon.

L'analyse HPLC-UV qui permet de quantifier et d'identifier la théanine contenue dans les différents extraits de thé obtenus selon le procédé de l'invention ou selon un procédé dit conventionnel, montre que les extraits de thé obtenus avec le procédé de l'invention contiennent de la théanine. Cependant on observe moins de théanine dans les extraits obtenus selon l'invention que par l'extraction conventionnelle tels que présentés sur la [fig. 4].

La caractérisation de la théine qui est la même molécule que la caféine, est un alcaloïde présent en grande quantité dans les feuilles de thé. La théine contenue dans les extraits de thés selon l'invention versus les extraits de thés conventionnels a également été déterminée. Tous les échantillons ont été séparés sur une colonne Uptisphere CS evolution C18-AQ de 100 mm x 4,6 mm x 2,6 pm (Interchim) par un système HPLC Agilent 1100 (Agilent Technologies). Le débit était de 0,8 ml/min. La phase mobile était constituée de 0,1 % de TFA (acides trifluoroacétiques) en solution aqueuse (A) et de méthanol (B). L'élution a été facilitée par le programme de gradient comme suit, voir tableau 6 :

**[Tableau 6]**

| Temps (min) | A eau/0,1 % TFA (%) | B Méthanol (%) |
|---|---|---|
| 0 | 100 | 0 |
| 17,5 | 79 | 21 |
| 18,8 | 0 | 100 |
| 21 | 0 | 100 |
| 22 | 100 | 0 |

La température de la colonne a été maintenue à 25° C. Le volume d'injection était de 5 µL et la longueur d'onde de détection a été réglée à 272 nm en utilisant un détecteur UV. L'étalon de théine a été acheté chez Sigma-Aldrich. L'identification de la théine a été réalisée par comparaison des temps de rétention et des pics spectraux UV de l'échantillon avec un standard authentique. Une estimation quantitative de la théine a été effectuée sur la base de l'aire des pics chromatographiques comparée à l'air de l'étalon.

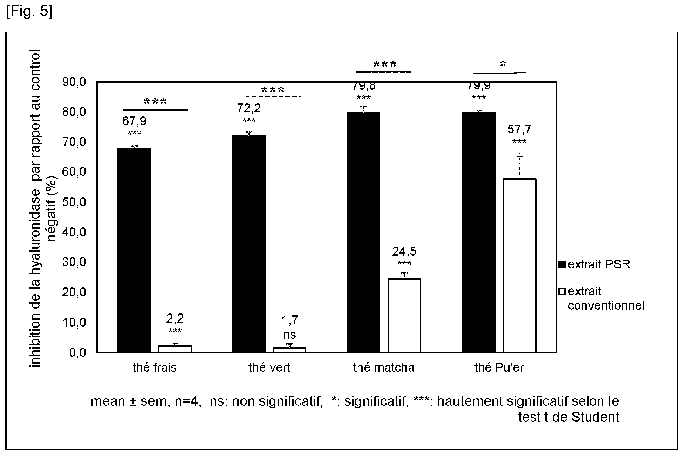

L'analyse HPLC-UV qui permet de quantifier et d'identifier la théine contenue dans les différents extraits de thé obtenus selon le procédé de l'invention ou selon un procédé dit conventionnel, montre que tous les types d'extraits de thé obtenus avec le procédé de l'invention ou selon une extraction dite conventionnelle contiennent de la théine. Les résultats montrent que les extraits obtenus selon le procédé de l'invention contiennent moins de théine que les extraits de thé obtenus par l'extraction conventionnelle tels que présentés sur la [fig. 5].

La quantification de la catéchine a également été réalisée. La catéchine existe sous forme de plusieurs stéréo-isomères due à la présence de deux carbones asymétriques. Dans la nature, les isomères les plus fréquents sont la (+)-catéchine et la (-)-épicatéchine. Les deux autres énantiomères sont beaucoup plus rares et leur présence semble liée à des réactions enzymatiques ou à des traitements thermiques. Tous les échantillons ont été séparés sur une colonne Uptisphere CS evolution C18-AQ de 100 mm x 4,6 mm x 2,6 pm (Interchim) par un système HPLC Agilent 1100 (Agilent Technologies). Le débit était de 0,8 ml/min. La phase mobile était constituée de 0,1 % de TFA (acides trifluoroacétiques) en solution aqueuse (A) et de méthanol (B). L'élution a été facilitée par le programme de gradient comme suit, voir tableau 7 :

**[Tableau 7]**

| Temps (min) | A eau/0,1 % TFA (%) | B Méthanol (%) |
|---|---|---|
| 0 | 100 | 0 |
| 4 | 100 | 0 |
| 20,5 | 65,9 | 54,1 |
| 27 | 32 | 68 |
| 29 | 5 | 95 |

La température de la colonne a été maintenue à 25°C. Le volume d'injection était de 20 µL et la longueur d'onde de détection a été réglée à 255 nm, 280 nm, 290 nm et 324 nm en utilisant un détecteur UV. Le standard de catéchine a été acheté auprès de Sigma-Aldrich. L'identification de la catéchine a été réalisée par comparaison des temps de rétention et des pics spectraux UV de l'échantillon avec un standard authentique. Une estimation quantitative de la catéchine a été effectuée sur la base de l'aire de pic des concentrations de l'échantillon par rapport à l'aire de l'étalon.

L'analyse HPLC-UV qui permet de quantifier et d'identifier la catéchine contenue dans les différents extraits de thé obtenus selon le procédé de l'invention ou selon un procédé dit conventionnel, montre que les extraits de thé obtenus soit par le procédé de l'invention soit par l'extraction conventionnelle contiennent peu voire pas de polyphénols type catéchine tel qu'illustré dans la [fig. 4]. Seuls les extraits obtenus à partir de thé pu'er contiennent un peu de catéchine. Cette absence ou la faible quantité de ces molécules dans les extraits s'explique notamment par la réalisation des étapes de traitement par les charbons et par le PVPP qui ont ôté ce type de composé. Ces résultats ont également été confirmés et complétés par la réalisation d'une chromatographie sur couche mince permettant de visualiser différentes molécules comme la catéchine, l'épicatéchine et l'epigallocatéchine. Aucun des extraits de thé qu'ils soient obtenus par le procédé de l'invention ou par le procédé conventionnel ne contenait ces molécules. Ceci confirmant l'efficacité du traitement charbon et PVPP.

La quantification et la mesure de la taille des ARN de bas poids moléculaires ont été réalisées par une technique d'électrophorèse miniaturisée sur des puces microfluidiques spécifiques de l'analyse des acides nucléiques telle que celle des ARN de petits poids moléculaires (Bioanalyseur 2100^{®}, Agilent). Cette méthode permet de déterminer la taille et la concentration en acides nucléiques contenues dans un extrait.

Les résultats de la quantification par le Bioanalyseur montrent que les extraits de thé obtenus par le procédé décrit dans la présente invention, permettent d'extraire les ARN de petits poids moléculaires à partir des différents types de thé tel qu'illustré sur le tableau 8. En revanche aucun ARN de petits poids moléculaires n'est détectable dans les extraits de thé conventionnel (nd).

**[Tableau 8]**

| Type d'extrait | Petits ARN (mg/kg) |
|---|---|
| PSR thé frais | 74 |
| Thé frais conventionnel | nd |
| PSR thé vert | 77 |
| Thé vert conventionnel | nd |
| PSR thé matcha | 61 |
| Thé matcha conventionnel | nd |
| PSR thé pu'er | 177 |
| Thé pu'er conventionnel | nd |
| nd : non détecté | |

Evaluation des extraits de thé des exemples 1 et 2 sur leur activité vis-à-vis de l'inhibition d'une enzyme, la hyaluronidase en test *in vitro :*

### Principe :

L'objectif de cet essai est de démontrer le pouvoir inhibiteur des extraits issus des exemples 1 et 2 sur l'activité d'une enzyme telle que la hyaluronidase via un test mesurant la turbidité. La hyaluronidase catalyse la dégradation de l'acide hyaluronique (un glycosaminoglycane fortement présent au niveau du derme de la peau ayant des propriétés hydratantes et anti-âge) en mono ou disaccharides ainsi qu'en fragments plus petits d'acide hyaluronique. L'acide hyaluronique a la capacité de réagir avec une solution d'albumine acide et ainsi de former un trouble qui peut être mesuré au spectrophotomètre à 600 nm. La mesure de l'inhibition de l'activité enzymatique se fait donc en analysant les niveaux de turbidité des échantillons après les avoir mis en contact avec l'enzyme et son substrat.

### Protocole :

L'enzyme est incubée avec les extraits obtenus selon les exemples 1 et 2, de manière à représenter une concentration finale de 1 % dans le mélange réactionnel, pendant 20 minutes à une température de 37°C, dans un tampon à pH7, conditions idéales pour l'équilibre enzymatique. Le substrat de l'enzyme, l'acide hyaluronique est ajouté au mélange à une concentration de 0,3 mg/ml. L'ensemble est homogénéisé lentement puis incubé pendant exactement 45 minutes à 37°C. L'acide hyaluronique restant dans le mélange est ensuite mis en contact avec une solution d'albumine acide et homogénéisé. Le temps laissé à la réaction est de 10 minutes avant que la transmittance ne soit lue à 600 nm au spectrophotomètre. Les données sont comparées à celles obtenues avec un contrôle négatif d'inhibition correspondant à la condition où l'enzyme a dégradé à 100 % le substrat, correspondant ainsi à une activité enzymatique de 100%.

### Résultats :

Les extraits de thé préparés selon l'exemple 1 montrent un pourcentage d'inhibition de la hyaluronidase allant de 67,9 % (pour le faible, le thé frais) à environ 80 % (pour les plus forts, le thé matcha et pu'er). Les extraits de thé préparés de manière conventionnelle selon l'exemple 2 montrent des pourcentages d'inhibition allant de 1,7 % (le plus faible, le thé vert) à 57,7 % (le plus fort, le thé pu'er).

### Conclusion :

Les essais d'évaluation de l'effet inhibiteur d'extraits sur l'activité de la hyaluronidase *in vitro* ont démontré que les extraits de thé préparés selon l'exemple 1 présentent un pouvoir inhibiteur de l'enzyme significativement plus fort que les extraits préparés selon l'exemple 2 tels que montrés en [fig. 5].

Exemple 6 : Evaluation de l'extrait de thé pu'er préparé selon l'exemple 1 versus l'extrait de thé pu'er conventionnel préparé selon l'exemple 2 sur les espèces oxygénées réactives après application d'un stress au glutamate monosodique sur des biopsies de peau

### Principe :

Le but de cette étude est de montrer l'effet de l'extrait de thé pu'er préparé selon l'invention sur la diminution des espèces oxygénées réactives générées par un stress au glutamate monosodique. Le glutamate est connu comme un neurotransmetteur excitateur du système nerveux central. Dans la peau, la signalisation du glutamate a lieu dans les terminaisons nerveuses nociceptives et les fibres mécanoréceptrices.

La thiorédoxine (TRX) est une classe de petites protéines impliquées dans les phénomènes d'oxydo-réduction connues pour être présentes dans tous les organismes. La fonction principale de la protéine interagissant avec la TRX (TXNIP) est de lier et d'inactiver TRX, ce qui entraîne une augmentation de l'accumulation des espèces oxygénées réactives et de l'apoptose. L'expression de TXNIP est en corrélation robuste avec le niveau de production des espèces oxygénées réactives.

### Protocole :

Des biopsies de peau humaine ex vivo sont mises en culture avec un milieu contenant du glutamate monosodique à 100 mM et traitées soit avec du PBS (tampon phosphate salin) pour le contrôle stressé soit avec l'extrait de thé pu'er préparé selon l'exemple 1 à 0,5 % (dilution volume/volume) et 1 % (dilution volume/volume) soit avec l'extrait de thé pu'er conventionnel préparé selon l'exemple 2 aux mêmes concentrations pendant 48 heures. En contrôle non stressé, des biopsies de peau sont mises en culture avec un milieu classique et traitées avec du PBS. Après traitement, les biopsies sont fixées pour analyse histologique et incluses en paraffine. Après déparaffinage, les sites antigéniques sont démasqués par chaleur au micro-onde dans du tampon citrate à pH=6 et une saturation est faite avec de l'albumine sérique bovine à 5 % pendant 30 minutes. Un anticorps monoclonal de lapin anti-TXNIP est ensuite appliqué au 1/100e pendant 1 heure et 30 minutes en chambre humide. Les coupes sont rincées dans un tampon phosphate puis incubées avec un anticorps secondaire Alexa 488^{®} anti-lapin pendant 1 heure en chambre humide, à l'abri de la lumière. Finalement les coupes sont à nouveau rincées dans un tampon phosphate puis les lames sont montées pour l'examen au microscope Eclipse E600 (Nikon). Les photos sont prises avec la camera Qlmaging Retiga 2000R Fast1394 et analysées par le logiciel Q-Capture Pro 7 (Qlmaging).

### Résultats :

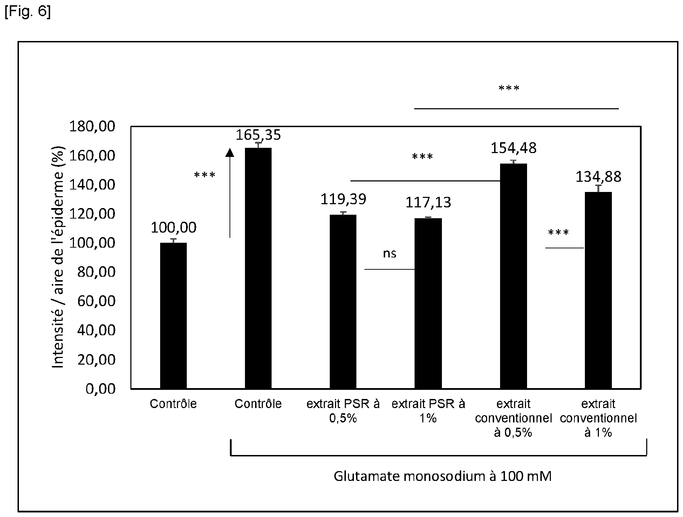

L'application du stress au glutamate monosodique a entrainé une augmentation des espèces réactives de l'oxygène (EROs) de +65 % par rapport aux biopsies de peau non stressées. Lorsque les biopsies de peau ont été traitées par l'extrait de l'invention à 0,5 % (dilution volume/volume) et à 1 % (dilution volume/volume), les EROs sont diminuées de façon hautement significative de -28 % et -29 % respectivement par rapport aux explants stressés non traités. Un extrait conventionnel de thé pu'er testé dans les mêmes conditions a permis une diminution plus faible de -7 % et -18 % respectivement. Les résultats sont présentés en [fig. 6]. Moyenne +/- sem; n=10. Analyses statistiques avec le test-t de Student, ns : non significatif ; *** : hautement significatif.

### Conclusion :

L'extrait de thé pu'er préparé selon l'invention a montré une activité antioxydante, dirigée contre les espèces réactives de l'oxygène. Cette activité s'est révélée supérieure à celle obtenue avec un extrait conventionnel de thé pu'er.

### Exemple 7 : Formule d'une crème riche

**[Tableau 9]**

| Ingrédients (Nom de marque) | | INCI | % w/w |
|---|---|---|---|
| Phase A | | | |
| Eau purifiée | Aqua | | Qsp 100 100 |
| Optiphen^{™} Plus preservative | Phenoxyethanol (and) Caprylyl Glycol (and) Sorbic Acid | | 1,50 |
| Phase B | | | |
| Stabileze^{™} QM polymer | PVM/MA Decadiene Crosspolymer | | 0,15 |
| Phase C | | | |
| ProLipid^{™} 141 lamellar gel | Glyceryl Stearate (and) Behenyl Alcohol (and) Palmitic Acid (and) Stearic Acid (and) Lecithin (and) Lauryl Alcohol (and) Myristyl Alcohol (and) Cetyl Alcohol | | 5,00 |
| Ceraphyl^{™} 494 ester | Isocetyl Stearate | | 4,00 |
| Ceraphyl^{™} SLK ester | Isodecyl Neopentanoate | | 4,00 |
| Dowsil 580 Wax | Stearoxytrimethylsilane (and) Stearyl Alcohol | | 2,00 |
| Emulsynt^{™} GDL ester | Glyceryl Dilaurate | | 3,00 |
| Phase D | | | |
| Gransil DM-5 | Dimethicone (and) Polysilicone-11 | | 3,00 |
| Phase E | | | |
| Hydroxyde de sodium | Sodium Hydroxide | | 0,04 |
| Eau purifiée | Aqua | | 0,50 |
| Phase F | | | |
| parfum | Parfum/Fragrance | | 0,30 |
| Phase G | | | |
| Extrait selon l'exemple 1 | Butylene glycol (and) *Camellia Sinensis* Leaf Extract | | 1,00 |
| Covabead Velvet 10 | Polymethyl Methacrylate | | 1,00 |
| Phase H | | | |
| Eau purifiée | Aqua | | 15,00 |
| Natrosol^{™} Plus 330 HMHEC | Cetyl Hydroxyethylcellulose | | 0,50 |

### Procédé de préparation :

1. Homogénéiser la phase A dans le récipient principal et commencer à chauffer à 75-80°C ;
2. A 30°C, saupoudrer dans la phase B et homogénéiser tout en chauffant ;
3. Dans un bécher à part, préparer la phase C, chauffer à 75-80°C jusqu'à homogénéité ;
4. A 75°C, ajouter la phase C dans le récipient principal et homogénéiser pendant 10 minutes ;
5. Laisser refroidir la température et ajouter la phase D à 65°C. Bien mélanger pour homogénéiser pendant 10 minutes ;
6. Prémélanger la phase E avant de l'ajouter dans le récipient principal ;
7. Ajouter la phase E à 60°C. Bien mélanger pour homogénéiser pendant 10 minutes ;
8. A 35°C, ajouter la phase F et bien mélanger ;
9. Prémélanger la phase G avant de l'ajouter dans le récipient principal ;
10. Ajouter la phase G à 35°C. Bien mélanger pour homogénéiser ;
11. Dans un bécher à part, préparer la phase H : saupoudrer Natrosol^{™} dans l'eau à température ambiante et homogénéiser tout en chauffant à 60°C ;
12. Ajouter la phase H à 30°C. Bien mélanger pour homogénéiser ;
13. Arrêter à 25°C.

La composition se présente ainsi sous forme d'une crème beurre blanche, avec un pH compris entre 4,90 et 5,40 et une viscosité (D0) de 160000 - 210000 cps (Brookfield RVT/Spindle D/5 RPM/1 minute/25°C).

### Exemple 8 : Formule d'un masque anti-âge

**[Tableau 10]**

| Ingrédients (Nom de marque) | | INCI | % w/w |
|---|---|---|---|
| Phase A | | | |
| Eau purifiée | Aqua | | Qsp 100 |
| EDTA tétrasodique | Tetrasodium EDTA | | 0,05 |
| Phase B | | | |
| N-Hance^{™} HP40S guar | Hydroxypropyl Guar | | 0,10 |
| Phase C | | | |
| Lubrajel^{™} DV Free hydrogel | Glycerin (and) Glyceryl Acrylate/Acrylic Acid Copolymer | | 6,00 |
| Phase D | | | |
| Si-Tec^{™} GF 3096 silicone | Dimethicone (and) Dimethiconol | | 12,00 |
| RapiThix^{™} A-60 polymer | Sodium Polyacrylate (and) Hydrogenated Polydecene (and) Trideceth-6 | | 2,40 |
| Phase E | | | |
| Optiphen^{™} Plus preservative | Phenoxyethanol (and) Caprylyl Glycol (and) Sorbic Acid | | 1,50 |
| Phase F | | | |
| Surfin* 96 | Alcohol Denat. | | 3,50 |
| parfum | Parfum/Fragrance | | 0,50 |
| Phase G | | | |
| Extrait selon l'exemple 1 | Water/Aqua (and) Butylene glycol (and) *Camellia Sinensis* Leaf Extract | | 1,00 |
| Xirona Carribean Blue | Mica (and) CI 77891 (Titanium Dioxide) (and) Silica (and) Tin Oxide | | 1,00 |

### Procédé de préparation :

1. A 25°C, homogénéiser la phase A dans le récipient principal ;
2. A 25°C, saupoudrer dans la phase B et bien mélanger jusqu'à homogénéité ;
3. A 25°C, ajouter la phase C et bien mélanger jusqu'à homogénéité ;
4. Prémélanger la phase D dans un bécher à part et ajouter dans le récipient principal à 25°C ;
5. A 25°C, ajouter la phase E dans le récipient principal et bien mélanger ;
6. Prémélanger la phase F et l'ajouter lentement. Bien mélanger jusqu'à homogénéité ;
7. Prémélanger la phase G dans un bécher à part et ajouter dans le récipient principal jusqu'à homogénéité ;
8. Arrêter à 25°C.

La composition se présente ainsi sous forme d'un gel crème avec des effets vert scintillant, avec un pH compris entre 5,30 et 5,80 et une viscosité (D0) de 70000 - 100000 cps (Brookfield RVT/Spindle C/5 RPM/1 minute/25°C).

### Exemple 9 : Formule d'un Sérum

**[Tableau 11]**

| Ingrédients (Nom de marque) | INCI | % w/w |
|---|---|---|
| Phase A | | |
| Eau déminéralisée | Aqua | 87,40 |
| Sodium Hyaluronate | Sodium Hyaluronate | 0,20 |
| RapiThix^{™} A-60 polymer | Sodium Polyacrylate (and) Hydrogenated Polydecene (and) Trideceth-6 | 0,40 |
| Lubrajel^{™} DV hydrogel | Glycerin (and) Glyceryl Acrylate/Acrylic Acid Copolymer (and) Propylene Glycol | 6,00 |
| Lubrajel^{™} Oil hydrogel | Glycerin (and) Glyceryl Acrylate/Acrylic Acid Copolymer (and) Propylene Glycol (and) PVM/MA Copolymer | 1,00 |
| Wacker-Belsil* DM 100 | Dimethicone | 2,00 |
| Cyclopentasiloxane NF | Cyclopentasiloxane | 0,50 |
| Extrait selon l'exemple 1 | Water/Aqua (and) Butylene glycol (and) *Camelia Sinensis* Leaf Extract | 1,00 |
| Optiphen^{™} preservative | Phenoxyethanol (and) Caprylyl Glycol | 1,50 |

### Procédé de préparation :

1. Ajouter de l'eau dans le récipient principal et commencer le mélange avec une pale d'hélice hi-lo ;
2. Ajouter le reste des ingrédients, l'un après l'autre tout en mélangeant entre chaque addition.

La composition se présente ainsi sous forme d'un sérum lisse, semi-opaque, avec un pH compris entre 5,75 et 6,25 et une viscosité (D0) de 1,100 - 1,400 cps (Brookfield RVT/spindle 3/20 rpm/25°C/1 minute).

## Revendications

1. Procédé d'obtention d'un extrait aqueux de feuilles de thé *(Camellia sinensis)* choisies parmi le thé frais, le thé pu'er, le thé matcha ou le thé vert, comprenant les étapes suivantes :
a) on met en présence les feuilles de thé ou la poudre de thé avec de l'eau, puis éventuellement on les broie ;
b) on ajoute de l'acide phytique ;
c) on ajuste le pH à une valeur comprise entre 10 et 11 ;
d) on maintien le mélange sous agitation pendant au moins une heure à une température comprise entre 40 et 80°C ;
e) on purifie le mélange obtenu en c) pour éliminer la matière végétale solide résiduelle et récolter le filtrat ;
f) on ajuste le pH à une valeur comprise entre 6 et 8 ;
g) on traite par du charbon actif en poudre ;
h) on filtre pour éliminer le charbon actif et on récolte le filtrat ;
i) on traite le filtrat à l'aide de polyvinylpolypyrrolidones (PVPP) ;
j) on filtre pour éliminer les polyvinylpolypyrrolidones et récolter le filtrat ;
k) on contrôle et réajuste le pH du filtrat, si nécessaire, à une valeur comprise entre 6 et 6,5.

2. Procédé selon la revendication 1, **caractérisé en ce qu'**à l'étape a) on met en présence les feuilles de thé avec de l'eau, dans un rapport matière végétale / eau de 3 % à 20 % en poids/poids et préférentiellement dans un rapport entre 3 % et 10 %.

3. Procédé selon l'une des revendications 1 ou 2, **caractérisé en ce qu'**à l'étape b) le traitement est réalisé avec de l'acide phytique à une concentration comprise entre 1 et 10 mM et préférentiellement entre 1 et 5 mM.

4. Procédé selon l'une des revendications 1 à 3, **caractérisé en ce qu'**à l'étape e) on réalise au moins une filtration sur des filtres de porosité supérieure ou égale à 30 µm.

5. Procédé selon l'une des revendications 1 à 4, **caractérisé en ce qu'**à l'étape j) on réalise des filtrations séquentielles avec des filtres de porosité supérieure à 25 µm, suivie d'une filtration avec un filtre de porosité 0,8 µm.

6. Procédé selon l'une des revendications 1 à 5, **caractérisé en ce qu'**après l'étape k) l'extrait est ultérieurement dilué dans un solvant physiologiquement acceptable puis que son pH est ajusté à une valeur comprise entre 5,8 et 6,5 préférentiellement entre 6,0 et 6,5, pour obtenir un extrait dilué ayant un poids sec compris entre 4 à 20 g/kg.

7. Procédé selon la revendication 6, **caractérisé en ce que** le solvant physiologiquement acceptable est choisi parmi l'eau, le glycérol, l'éthanol, le propanediol, le butylène glycol, le dipropylène glycol, les diglycols éthoxylés ou propoxylés, les polyols cycliques ou tout mélange de ces solvants.

8. Extrait brut aqueux de feuilles de thé obtenu à partir de thé frais, de thé pu'er, de thé matcha ou de thé vert, enrichi en petits ARN d'une longueur d'au maximum 150 nucléotides, en sucres, en composés phénoliques et en acides organiques, dépourvu d'ADN, susceptible d'être obtenu par le procédé de l'une des revendications 1 à 5, **caractérisé en ce qu'**il a un poids sec de 5 à 30 g/kg, et qu'il comprend de 0,5 à 10 g/kg de sucres, de 0,050 à 2 g/kg d'acides organiques, de 0,050 à 5 g/kg de composés phénoliques dont 0 à 200 mg/kg de catéchine, 0,020 à 2 g/kg de théine et 0,020 à 2 g/kg de théanine et de 50 à 350 mg/kg d'ARN de petit poids moléculaire d'une longueur d'au maximum 150 nucléotides.

9. Extrait aqueux dilué de feuilles de thé obtenu à partir de thé frais, de thé pu'er, de thé matcha ou de thé vert, enrichi en petits ARN d'une longueur d'au maximum 150 nucléotides, en sucres, en composés phénoliques et en acides organiques, dépourvu d'ADN, susceptible d'être obtenu par le procédé de l'une des revendications 6 ou 7, **caractérisé en ce qu'**il a un poids sec compris entre 4 à 20 g/kg de poids sec, une concentration de 0,2 à 5 g/kg de sucres, 0,030 à 1 g/kg d'acides organiques, 0,030 à 3 g/kg de composés phénoliques dont 0 à 120 mg/kg de catéchine, 0,010 à 1 g/kg de théine et 0,010 à 1 g/kg de théanine et 10 à 250 mg/kg d'ARN de petit poids moléculaire d'une longueur d'au maximum 150 nucléotides, en poids du poids total de l'extrait.

10. Composition comprenant, en tant qu'ingrédient actif, une quantité efficace d'au moins un extrait dilué selon la revendication 9, choisi parmi le thé frais, le thé pu'er, le thé matcha ou le thé vert ou tout mélange de ceux-ci, et un milieu physiologiquement acceptable.

11. Composition selon la revendication 10, dans laquelle ledit ingrédient actif est présent à une concentration comprise entre 0,05 et 5 % en poids du poids total de la composition et préférentiellement entre 0,1 à 2,5 % en poids du poids total de la composition et un milieu physiologiquement acceptable.

12. Composition selon l'une des revendications 10 ou 11, **caractérisée en ce qu'**elle est formulée pour être appliquée topiquement sur la peau, les phanères et le cuir chevelu.

13. Utilisation cosmétique non thérapeutique de la composition de l'une des revendications 10 à 12 pour le soin de la peau, du cuir chevelu et des phanères et plus particulièrement pour protéger la peau des agressions externes et de l'oxydation, lutter contre les signes du vieillissement cutané, éclaircir la peau, ou encore améliorer l'hydratation de la peau.

14. Composition selon l'une des revendications 10 à 12 pour son utilisation pour augmenter la photoprotection de la peau.

## Patentansprüche

1. Verfahren zur Gewinnung eines wässrigen Extrakts aus Teeblättern *(Camellia sinensis),* die aus frischem Tee, Pu-Erh-Tee, Matcha-Tee oder grünem Tee ausgewählt sind, wobei es die folgenden Schritte umfasst:
a) Zusammenbringen der Teeblätter oder des Teepulvers mit Wasser, woraufhin diese möglicherweise zerkleinert werden;
b) Zusetzen von Phytinsäure;
c) Einstellen des pH auf einen Wert im Bereich von 10 bis 11;
d) fortwährendes Rühren der Mischung über einen Zeitraum von mindestens einer Stunde bei einer Temperatur im Bereich von 40 und 80 °C;
e) Aufreinigen der in c) erhaltenen Mischung, um die verbleibenden pflanzlichen Feststoffe zu entfernen und das Filtrat aufzufangen;
f) Einstellen des pH auf einen Wert im Bereich von 6 bis 8;
g) Behandeln mit pulverförmiger Aktivkohle;
h) Filtrieren, um die Aktivkohle zu entfernen, und Auffangen des Filtrats;
i) Behandeln des Filtrats mit Hilfe von Polyvinylpolypyrrolidonen (PVPP);
j) Filtrieren, um die Polyvinylpolypyrrolidone zu entfernen und das Filtrat aufzufangen;
k) Überprüfen und erforderlichenfalls erneutes Einstellen des pH auf einen Wert im Bereich von 6 bis 6,5.

2. Verfahren nach Anspruch 1, **dadurch gekennzeichnet, dass** die Teeblätter in Schritt a) mit Wasser zusammengebracht werden, in einem Verhältnis von Pflanzenmaterial / Wasser, das 3 % bis 20 % nach Gewicht/Gewicht beträgt, und vorzugsweise in einem Verhältnis zwischen 3 % und 10 %.

3. Verfahren nach einem der Ansprüche 1 oder 2, **dadurch gekennzeichnet, dass** in Schritt b) die Behandlung mit Phytinsäure in einer Konzentration durchgeführt wird, welche im Bereich von 1 bis 10 mM und vorzugsweise von 1 bis 5 mM liegt.

4. Verfahren nach einem der Ansprüche 1 bis 3, **dadurch gekennzeichnet, dass** in Schritt e) mindestens eine Filtration über Filter vorgenommen wird, deren Porenweite mindestens 30 µm beträgt.

5. Verfahren nach einem der Ansprüche 1 bis 4, **dadurch gekennzeichnet, dass** in Schritt j) eine Reihe von Filtrationen mit Filtern vorgenommen wird, deren Porenweite mehr als 25 µm beträgt, gefolgt von einer Filtration mit einem Filter, dessen Porenweite 0,8 µm beträgt.

6. Verfahren nach einem der Ansprüche 1 bis 5, **dadurch gekennzeichnet, dass** nach Schritt k) der Extrakt später in einem physiologisch unbedenklichen Lösungsmittel verdünnt wird, woraufhin dessen pH auf einen Wert im Bereich von 5,8 bis 6,5, vorzugsweise zwischen 6,0 und 6,5 eingestellt wird, um einen verdünnten Extrakt mit einem Trockengewicht im Bereich von 4 bis 20 g/kg zu erhalten.

7. Verfahren nach Anspruch 6, **dadurch gekennzeichnet, dass** das physiologisch unbedenkliche Lösungsmittel aus Wasser, Glycerin, Ethanol, Propandiol, Butylenglykol, Dipropylenglykol, ethoxylierten oder propoxylierten Diglykolen, zyklischen Polyolen oder einer beliebigen Mischung dieser Lösungsmittel ausgewählt ist.

8. Wässriger Rohextrakt aus Teeblättern, der ausgehend von frischem Tee, Pu-Erh-Tee, Matcha-Tee oder grünem Tee erhalten wurde, angereichert mit kleinen RNA, deren Länge höchstens 150 Nukleotide beträgt, mit Zuckerarten, mit phenolischen Verbindungen und mit organischen Säuren, frei von DNA, wobei er durch das Verfahren nach einem der Ansprüche 1 bis 5 erhalten werden kann, **dadurch gekennzeichnet, dass** er ein Trockengewicht von 5 bis 30 g/kg hat und dass er 0,5 bis 10 g/kg an Zuckerarten, 0,050 bis 2 g/kg an organischen Säuren, 0,050 bis 5 g/kg an phenolischen Verbindungen, davon 0 bis 200 mg/kg an Catechin, 0,020 bis 2 g/kg an Thein und 0,020 bis 2 g/kg an Theanin, und 50 bis 350 mg/kg an niedermolekularer RNA mit einer Länge von höchstens 150 Nukleotiden umfasst.

9. Wässriger Rohextrakt aus Teeblättern, der ausgehend von frischem Tee, Pu-Erh-Tee, Matcha-Tee oder grünem Tee erhalten wurde, angereichert mit kleinen RNA, deren Länge höchstens 150 Nukleotide beträgt, mit Zuckerarten, mit phenolischen Verbindungen und mit organischen Säuren, frei von DNA, wobei er durch das Verfahren nach einem der Ansprüche 6 oder 7 erhalten werden kann, **dadurch gekennzeichnet, dass** er ein Trockengewicht von 4 bis 20 g/kg, eine Konzentration von 0,2 bis 5 g/kg an Zuckerarten, 0,030 bis 1 g/kg an organischen Säuren, 0,030 bis 3 g/kg an phenolischen Verbindungen, davon 0 bis 120 mg/kg an Catechin, 0,010 bis 1 g/kg an Thein und 0,010 bis 1 g/kg an Theanin, und 10 bis 250 mg/kg an niedermolekularer RNA mit einer Länge von höchstens 150 Nukleotiden aufweist, nach Gewicht vom Gesamtgewicht des Extrakts.

10. Zusammensetzung, die als Wirkbestandteil eine wirksame Menge mindestens eines verdünnten Extrakts nach Anspruch 9, wobei dieser aus frischem Tee, Pu-Erh-Tee, Matcha-Tee oder grünem Tee oder einer beliebigen Mischung davon ausgewählt ist, sowie ein physiologisch unbedenkliches Medium umfasst.

11. Zusammensetzung nach Anspruch 10, wobei der Wirkbestandteil in einer Konzentration im Bereich von 0,05 bis 5 % nach Gewicht vom Gesamtgewicht der Zusammensetzung vorzugsweise von 0,1 bis 2,5 % nach Gewicht vom Gesamtgewicht der Zusammensetzung vorliegt, und ein physiologisch unbedenkliches Medium.

12. Zusammensetzung nach einem der Ansprüche 10 bis 11, **dadurch gekennzeichnet, dass** sie dafür formuliert ist, topisch auf die Haut, die Hautanhangsgebilde und die Kopfhaut aufgebracht zu werden.

13. Nicht-therapeutische kosmetische Verwendung der Zusammensetzung nach einem der Ansprüche 10 bis 12, zur Pflege der Haut, der Kopfhaut und der Hautanhangsgebilde, und insbesondere zum Schutz der Haut vor äußeren schädlichen Einflüssen und Oxidation, zur Bekämpfung von Anzeichen der Hautalterung, zur Aufhellung der Haut, oder auch zur Verbesserung der Hydratisierung der Haut.

14. Zusammensetzung nach einem der Ansprüche 10 bis 12, zur Verwendung derselben, um den Lichtschutz der Haut zu erhöhen.

## Claims

1. Process for obtaining an aqueous extract of tea leaves (Camellia sinensis) selected from fresh tea, pu'er tea, matcha tea or green tea, comprising the following steps :
a) tea leaves or tea powder are brought into contact with water, then optionally ground;
b) phytic acid is added;
c) adjust the pH to between 10 and 11 ;
d) the mixture is stirred for at least one hour at a temperature of between 40 and 80°C;
e) the mixture obtained in c) is purified to remove residual solid plant matter and the filtrate is collected;
f) adjust the pH to a value between 6 and 8;
g) treat with powdered activated carbon;
h) filter to remove activated carbon and collect filtrate;
i) treat filtrate with polyvinylpolypyrrolidones (PVPP);
j) filter to remove polyvinylpolypyrrolidones and collect filtrate;
k) the pH of the filtrate is checked and readjusted, if necessary, to a value between 6 and 6.5.

2. Process according to claim 1, **characterized in that** in step a) the tea leaves are brought into contact with water, in a botanical matter/water ratio of 3% to 20% w/w and preferably in a ratio of between 3% and 10%.

3. Process according to one of claims 1 or 2, **characterized in that** in step b) the treatment is carried out with phytic acid at a concentration of between 1 and 10 mM and preferably between 1 and 5 mM.

4. Process according to one of claims 1 to 3, **characterized in that** in step e) at least one filtration is carried out on filters with a porosity greater than or equal to 30 µm.

5. Process according to one of claims 1 to 4, **characterized in that** in step j) sequential filtrations are carried out with filters having a porosity greater than 25 µm, followed by filtration with a filter having a porosity of 0.8 µm.

6. Process according to one of claims 1 to 5, **characterized in that** after step k) the extract is subsequently diluted in a physiologically acceptable solvent and its pH is adjusted to a value between 5.8 and 6.5, preferably between 6.0 and 6.5, to obtain a diluted extract with a dry weight between 4 and 20 g/kg.

7. Process according to claim 6, **characterized in that** the physiologically acceptable solvent is selected from water, glycerol, ethanol, propanediol, butylene glycol, dipropylene glycol, ethoxylated or propoxylated diglycols, cyclic polyols or any mixture of these solvents.

8. Crude aqueous extract of tea leaves obtained from fresh tea, pu'er tea, matcha tea or green tea, enriched in small RNA of a length of at most 150 nucleotides, in sugars, in phenolic compounds and in organic acids, devoid of DNA, obtainable by the process of one of claims 1 to 5, **characterized in that** it has a dry weight of from 5 to 30 g/kg, and it comprises from 0.5 to 10 g/kg of sugars, from 0.050 to 2 g/kg of organic acids, from 0.050 to 5 g/kg of phenolic compounds including 0 to 200 mg/kg of catechin, 0.020 to 2 g/kg of theine and 0.020 to 2 g/kg of theanine and from 50 to 350 mg/kg of small molecular weight RNA of a length of at most 150 nucleotides.

9. Diluted aqueous extract of tea leaves obtained from fresh tea, pu'er tea, matcha tea or green tea, enriched in small RNA of a length of at most 150 nucleotides, in sugars, in phenolic compounds and in organic acids, devoid of DNA, obtainable by the process of one of claims 6 or 7, **characterized in that** it has a dry weight of between 4 and 20 g/kg of dry weight, a concentration of 0.2 to 5 g/kg of sugars, 0.030 to 1 g/kg of organic acids, 0.030 to 3 g/kg of phenolic compounds including 0 to 120 mg/kg of catechin, 0.010 to 1 g/kg of theine and 0.010 to 1 g/kg of theanine and 10 to 250 mg/kg of small molecular weight RNA of a length of at most 150 nucleotides, by weight of the total weight of the extract.

10. Composition comprising, as active ingredient, an effective amount of at least one diluted extract according to claim 9, selected from fresh tea, pu'er tea, matcha tea or green tea or any mixture thereof, and a physiologically acceptable medium.

11. Composition according to claim 10, wherein said active ingredient is present at a concentration of between 0.05 and 5% by weight of the total weight of the composition and preferably between 0.1 and 2.5% by weight of the total weight of the composition and a physiologically acceptable medium.

12. Composition according to one of claims 10 or 11, **characterized in that** it is formulated to be applied topically to the skin, appendages and scalp.

13. Non-therapeutic cosmetic use of the composition of one of claims 10 to 12 for caring for the skin, scalp and appendages and more particularly for protecting the skin from external aggression and oxidation, combating the signs of skin ageing, lightening the skin or improving skin hydration.

14. Composition according to one of claims 10 to 12 for use in increasing photoprotection of the skin.
